# EUROPEAN PATENT APPLICATION

(11) **EP 1 857 447 A1**
(43) Date of publication of application: **21.11.2007**
(21) Application number: 06712431.3
(22) Date of filing: 26.01.2006
(51) Int. Cl.: C07D 263/58, A61K 31/423, A61K 31/428, A61P 3/06, A61P 3/10, A61P 9/00, A61P 9/10, A61P 29/00, A61P 43/00, C07D 277/82

(54) **PPAR-ACTIVATING COMPOUND**

(30) Priority: 27.01.2005 US 647014 P
(71) Applicant: Kowa Company, Ltd., Naka-ku Nagoya-shi, Aichi-ken 460-8625 (JP)
(72) Inventor: YAMAZAKI, Yukiyoshi, Tokyo, 1890014 (JP); TOMA, Tsutomu, Tokyo, 1870032 (JP); NISHIKAWA, Masahiro, Aichi, 4630070 (JP); YAMADA, Hajime, Tokyo, 1890022 (JP); OZAWA, Hidefumi, Tokyo 1930835 (JP); OKUDA, Ayumu, Tokyo, 1890022 (JP); ABE, Kazutoyo, Tokyo, 1810013 (JP)
(74) Representative: Hartz, Nikolai
(86) International application number: PCT/JP2006/301249
(87) International publication number: WO 2006/080407

(57) **Abstract**

The present invention provides a compound selectively activating PPARα, which is useful as a medicament. The present invention is specifically directed to a benzoic acid derivative represented by the following general formula (1): 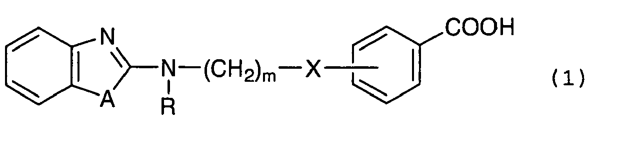 wherein A represents an oxygen atom, a nitrogen atom, or a sulfur atom; R represents a hydrogen atom, an alkyl group, an alkenyl group, an alkynyl group, a cycloalkyl group, a cycloalkyl-alkyl group, an aryl group, an aryl-alkyl group an aryl-oxy-alkyl group, a pyridyl-alkyl group, an alkoxycarbonyl-alkyl group, or a carboxy-alkyl group; X represents an oxygen atom, an NH group, or an S(O)ₙ group (wherein n represents an integer of 0, 1, or 2) ; and m represents an integer from 2 to 8; or a salt thereof. The present invention also provides a medicament comprising the benzoic acid derivative or the salt thereof as an active ingredient.

## Description

### Field of the Invention

The present invention relates to a PPAR-activating compound able to selectively activate a peroxisome proliferator-activated receptor α (PPARα) from among peroxisome proliferator-activated receptors (PPAR), which is useful as a medicament.

### Description of the Related Art

PPAR is known as one of nuclear receptor families and is hitherto known to have three subtypes (α, γ, and δ) (Non-patent documents 1 to 5).

Of these subtypes, PPARα is mainly expressed in the liver and is shown to be activated by plasticizers and fibrate drugs, for example, Wy14643 and commercially available medicaments such as clofibrate, fenofibrate, bezafibrate, andgemfibrozil (Non-patent documents 6 to 7).

PPARα activation is known to accelerate fatty acid β-oxidation in mammals, leading to decrease triglyceride in the blood. In humans, PPARα activation decreases lipids in the blood such as low density lipoprotein (LDL) cholesterol and very low density lipoprotein (VLDL) cholesterol, and PPARα-activating drugs are useful as preventive or therapeutic agents for hyperlipidemia and so on. Moreover, the PPARα-activating drugs increase high density lipoprotein (HDL) cholesterol and suppress the expression of VCAM-1, a cell adhesion molecule, in blood vessels, and as such, are also considered to be useful as preventive or therapeutic agents for arteriosclerosis and so on. Additionally, the PPARα-activating drugs are also considered to be useful in the prevention or treatment of diabetes and inflammatory diseases, in addition to cardiac diseases (Non-patent documents 8 to 14).

On the other hand, PPARγ is mainly expressed in adipocytes and is known to play an important role in the differentiation and proliferation of adipocytes. Thiazolidine derivatives, for example, drugs such as troglitazone, pioglitazone, and rosiglitazone, are known as PPARγ activators. These drugs have been reported to improve insulin resistance by inducing smaller, highly insulin-sensitive in adipocytes to replace fully-differentiated adipocytes with reduced insulin sensitivity (Non-patent documents 15 to 18). However, these drugs have also been reported to have unfavorable effects of causing weight gains and obesity attributed to increased fats in humans (Non-patent document 19). Recent reports indicate that PPARγ antagonists are also likely to improve insulin resistance (Non-patent documents 20 to 22).

Alternatively, PPARδ is ubiquitously present in body and has been reported to participate in lipid metabolism. However, there have been only a few reports about highly selective PPARδ activators, and the biological significance of PPARδ has remained unclear. Currently, the structures of PPARδ activators have been reported in many bibliographies (Non-patent documents 23, 24) . According to a recent report, GW501516, a PPARδ activator, elevates HDL levels in monkeys (Non-patent document 25) . Moreover, it has also been reported that activated PPARδ expressed in adipocytes or skeletal muscle cells promotes fat burning (Non-patent document 26). Meanwhile, compound F disclosed as a PPARδ activator in patent document 1 has been reported to have unfavorable effects of promoting lipid accumulation in human macrophages (Non-patent document 27). In addition, experiments using PPARδ-deficient mice suggest that PPARδ activation contributes to lipid-accumulating effects (Non-patent document 28). These phenomena are seen as mutually contradictory effects in the progression and treatment of arteriosclerosis. Accordingly, the therapeutic significance of PPARδ can be said to remain unclear.

From the viewpoint of the above, PPARα-selective activators that minimally activate PPARγ and PPARδ are expected to be useful in the prevention and treatment of hyperlipidemia, arteriosclerosis, diabetes, diabetes complications, inflammation, cardiac diseases, and so on, without weight gains and obesity.

Although some compounds activating PPARα have recently been reported (Patent documents 2 to 4), these compounds cannot be said to be PPARα-selective. Under the present circumstances, there remains unfound about highly PPARα-selective compounds useful in the prevention or treatment of the diseases described above.
Patent document 1 : WO 97/28149
Patent document 2 : WO 02/046176
Patent document 3 : WO 04/000762
Patent document 4 : WO 04/092130
Non-patent document 1 : Nature, 347, 645-650, 1990
Non-patent document 2 : Cell, 68, pp 879-887, 1992
Non-patent document 3 : Cell, 97, pp 161-163, 1999
Non-patent document 4 : Biochim. Biophys. Acta., 1302, pp 93-109, 1996
Non-patent document 5 : Journal of Medicinal Chemistry, 43, pp 527-550, 2000
Non-patent document 6 : Journal of the National Cancer Institute, 90, 1702-1709, 1998
Non-patent document 7 : Current Opinion in Lipidology, 10, pp 245-257, 1999
Non-patent document 8 : Journal of Atherosclerosis and Thrombosis, 3, pp 81-89, 1996
Non-patent document 9 : Current Pharmaceutical Design, 3, pp 1-14, 1997
Non-patent document 10 : Current Opinion in Lipidology, 10, pp 151-159, 1999
Non-patent document 11 : Current Opinion in Lipidology, 10, pp 245-257, 1999
Non-patent document 12 : The Lancet, 354, pp 141-148, 1999
Non-patent document 13 : Journal of Medicinal Chemistry, 43, pp 527-550, 2000
Non-patent document 14 : Journal of Cardiovascular Risk, 8, pp 195-201, 2001
Non-patent document 15 : Journal of Biological Chemistry, 270, 12953-12956, 1995
Non-patent document 16 : Endocrinology, 137, pp 4189-4195, 1996
Non-patent document 17 : Trends Endocrinol. Metab., 10, pp 9-13, 1999
Non-patent document 18 : J. Clin. Invest., 101, pp 1354-1361, 1998
Non-patent document 19 : The Lancet, 349, pp 952, 1997
Non-patent document 20 : Proc. Natl. Acad. Sci., 96, pp 6102-6106, 1999
Non-patent document 21 : The Journal of Biological Chemistry, 275, pp 1873-1877, 2000
Non-patent document 22 : J. Clin. Invest., 108, 1001-1013, 2001
Non-patent document 23 : Diabetes, 46, 1319-1327, 1997
Non-patent document 24 : Journal of Medicinal Chemistry, 43, pp 527-550, 2000
Non-patent document 25 : Proc. Natl. Acad. Sci., 98, pp 5306-5311, 2001
Non-patent document 26 : Cell, 113, pp 159-170, 2003
Non-patent document 27 : Journal of Biological Chemistry, 276, pp 44258-44265, 2001
Non-patent document 28 : Proc. Natl. Acad. Sci., 99, pp 303-308, 2002

### Disclosure of the Invention

### Problems to be solved by the Invention

An obj ect of the present invention is to provide a compound selectively activating PPARα, which is useful as a medicament.

### Means for solving the problem

The present inventors have conducted various studies and have consequently completed the present invention by finding out that a benzoic acid derivative or a sal t thereof represented by the formula (1) described below selectively activates PPARα and is useful as a preventive or therapeutic agent for hyperlipidemia, arteriosclerosis, diabetes, diabetes complications, inflammation, cardiac diseases, and so on, without weight gains and obesity.

That is, the present invention relates to a benzoic acid derivative represented by the following general formula (1) :

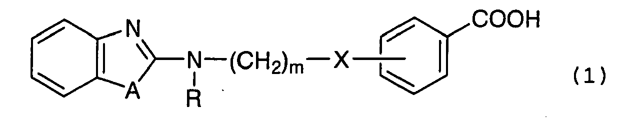

wherein A represents an oxygen atom, a nitrogen atom, or a sulfur atom;
R represents a hydrogen atom, a C₁₋₈ alkyl group, a C₃₋₈ alkenyl group, a C₃₋₈ alkynyl group, a C₃₋₇ cycloalkyl group, a C₃₋₇ cycloalkyl-C₁₋₈ alkyl group, a C₆₋₁₀ aryl group, a C₆₋₁₀ aryl-C₁₋₈ alkyl group (wherein the C₆₋₁₀ aryl moiety may be substituted with one or two selected from a halogen atom, a hydroxy group, a nitro group, an amino group, a di-C₁₋₄ alkylamino group, a C₁₋₄ alkyl group, a C₁₋₄ alkoxy group, a benzyloxy group, a phenylsulfonylmethyl group, and a C₁₋₄ alkanesulfonyloxy group), a C₆₋₁₀ aryl-oxy-C₁₋₈ alkyl group (wherein the C₆₋₁₀ aryl moiety may be substituted with one or two selected from a halogen atom, a hydroxy group, a nitro group, an amino group, a di-C₁₋₄ alkylamino group, a C₁₋₄ alkyl group, a C₁₋₄ alkoxy group, a benzyloxy group, a phenylsulfonylmethyl group, and a C₁₋₄ alkanesulfonyloxy group), a pyridyl-C₁₋₈ alkyl group, a C₁₋₈ alkoxycarbonyl-C₁₋₈ alkyl group, or a carboxy-C₁₋₈ alkyl group;
X represents an oxygen atom, an NH group, or an S(O)ₙ group (wherein n represents an integer of 0, 1, or 2); and
m represents an integer from 2 to 8; or a salt thereof.

The present invention also relates to a compound selected from 3-[3-[N-(Benzoxazol-2-yl)-N-3-(4-chlorophenoxy) ethyl] aminopropoxy] benzoic acid, 2- [5- [N- (Benzoxazol-2-yl) -N-isopropyl]aminopentylthio]benzoic acid, 2-[5-[N-(Benzoxazol-2-yl)-N-n-butyl]aminopentylthio]benzoic acid, 2-[5-[N-(Benzoxazol-2-yl)-N-n-pentyl]aminopentylthio]benzoic acid, and salts thereof.

The present invention also relates to a medicament comprising the benzoic acid derivative or the salt thereof as an active ingredient.

The present invention also relates to a preventive or therapeutic agent for hyperlipidemia comprising the benzoic acid derivative or the salt thereof as an active ingredient.

The present invention further relates to a preventive or therapeutic agent for arteriosclerosis comprising the benzoic acid derivative or the salt thereof as an active ingredient.

The present invention still further relates to a preventive or therapeutic agent for diabetes comprising the benzoic acid derivative or the salt thereof as an active ingredient.

The present invention relates to a preventive or therapeutic agent for diabetes complications comprising the benzoic acid derivative or the salt thereof as an active ingredient.

The present invention also relates to a preventive or therapeutic agent for inflammation comprising the benzoic acid derivative or the salt thereof as an active ingredient.

The present invention further relates to a preventive or therapeutic agent for cardiac diseases comprising the benzoic acid derivative or the salt thereof as an active ingredient.

The present invention still further relates to a pharmaceutical composition comprising the benzoic acid derivative or the salt thereof and a pharmaceutically acceptable carrier.

The present invention yet further relates to use of the benzoic acid derivative or the salt thereof for the manufacture of a preventive or therapeutic agent for hyperlipidemia.

The present invention also relates to use of the benzoic acid derivative or the salt thereof for the manufacture of a preventive or therapeutic agent for arteriosclerosis.

The present invention further relates to use of the benzoic acid derivative or the salt thereof for the manufacture of a preventive or therapeutic agent for diabetes.

The present invention still further relates to use of the benzoic acid derivative or the salt thereof for the manufacture of a preventive or therapeutic agent for diabetes complications.

The present invention yet further relates to use of the benzoic acid derivative or the salt thereof for the manufacture of a preventive or therapeutic agent for inflammation.

The present invention also relates to use of the benzoic acid derivative or the salt thereof for the manufacture of a preventive or therapeutic agent for cardiac diseases.

### Effect of the Invention

The benzoic acid derivative or the salt thereof of the present invention represented by the formula (1) has the effect of selectively activating PPARα from among PPAR subtypes and is useful as a preventive or therapeutic agent for hyperlipidemia, arteriosclerosis, diabetes, diabetes complications, inflammation, cardiac diseases, and so on, without weight gains and obesity.

### Best mode for carrying out the Invention

In the general formula (1), a C₁₋₈ alkyl group represented by R includes linear and branched alkyl groups such as methyl, ethyl, n-propyl, isopropyl, n-butyl, isobutyl, sec-butyl, tert-butyl, n-pentyl, isopenty, n-hexyl, isohexyl, n-heptyl, and n-octyl groups. Of these groups, methyl, ethyl, n-propyl, isopropyl, n-butyl, n-pentyl, n-hexyl, n-heptyl, and n-octyl groups are particularly preferred.

A C₃₋₈ alkenyl group represented by R includes linear and branchedalkenyl groups such as vinyl , allyl, butenyl, pentenyl, and hexenyl groups.

A C₃₋₈ alkynyl group represented by R includes linear and branched alkynyl groups such as propargyl and butynyl groups.

A C₃₋₇ cycloalkyl group represented by R includes cyclopropyl, cyclobutyl, cyclopentyl, cyclohexyl, and cycloheptyl groups.

More preferable examples of a C₃₋₇ cycloalkyl- C₁₋₈ alkyl group represented by R include C₃₋₇ cycloalkyl-C₁₋₄ alkyl groups, concretely including cyclopropylmethyl, cyclopropylethyl, cyclohexylmethyl, cyclohexylethyl, and cyclohexylpropyl groups. Of these groups, cyclohexylmethyl groups are particularly preferred.

A C₆₋₁₀ aryl group represented by R includes phenyl and naphthyl groups. Of these groups, naphthyl groups are particularly preferred.

Preferable examples of a C₆₋₁₀ aryl-C₁₋₈ alkyl group represented by R include C₆₋₁₀ aryl-C₁₋₄ alkyl groups. Phenyl-C₁₋₄ alkyl and naphthyl-C₁₋₄ alkyl groups are more preferred. Concrete examples thereof include benzyl, phenethyl, phenylpropyl, phenylbutyl, naphthylmethyl, naphthylethyl, and naphthyl propyl groups. Of these groups, phenyl-C₁₋₄ alkyl groups are more preferred, and benzyl groups are particularly preferred.
The C₆₋₁₀ aryl moiety in the C₆₋₁₀ aryl-C₁₋₈ alkyl group may be substituted with one or two selected from a halogen atom, a hydroxy group, a nitro group, an amino group, a di-C₁₋₄ alkylamino group, a C₁₋₄ alkyl group, a C₁₋₄ alkoxy group, a benzyloxy group, a phenylsulfonylmethyl group, and a C₁₋₄ alkanesulfonyloxy group. In this context, the halogen atom includes chlorine, bromine, and fluorine atoms. The di-C₁₋₄ alkylamino group includes dimethylamino, diethylamino, and diisopropylamino groups. The C₁₋₄ alkyl group includes methyl, ethyl, and isopropyl groups. The C₁₋₄ alkoxy group includes methoxy, ethoxy, n-propoxy, isopropoxy, and butoxy groups. The C₁₋₄ alkanesulfonyloxy group includes methanesulfonyloxy, ethanesulfonyloxy, and propanesulfonyloxy groups. More preferably, these substituents on the aryl moiety are one or two selected from a halogen atom, a nitro group, and a C₁₋₄ alkyl group. The substituents are still more preferably a halogen atom, particularly preferably a chlorine atom.

Preferable examples of a C₆₋₁₀ aryl-oxy-C₁₋₈ alkyl group represented by R include C₆₋₁₀ aryl-oxy-C₁₋₄ alkyl groups. Phenyl-oxy-C₁₋₄ alkyl groups are more preferred. Concrete examples thereof include phenyloxymethyl, phenyloxyethyl, phenyloxypropyl, and phenyloxybutyl groups. Of these groups, phenyloxyethyl and phenyloxypropyl groups are particularly preferred.
The C₆₋₁₀ aryl moiety in the C₆₋₁₀ aryl-oxy-C₁₋₈ alkyl group may be substituted with one or two selected from a halogen atom, a hydroxy group, a nitro group, an amino group, a di-C₁₋₄ alkylamino group, a C₁₋₄ alkyl group, a C₁₋₄ alkoxy group, a benzyloxy group, a phenylsulfonylmethyl group, and a C₁₋₄ alkanesulfonyloxy group. In this context, the halogen atom includes chlorine, bromine, and fluorine atoms. The di-C₁₋₄ alkylamino group includes dimethylamino, diethylamino, and diisopropylamino groups. The C₁₋₄ alkyl group includes methyl, ethyl, and isopropyl groups. The C₁₋₄ alkoxy group includes methoxy, ethoxy, n-propoxy, isopropoxy, and butoxy groups. The C₁₋₄ alkanesulfonyloxy group includes methanesulfonyloxy, ethanesulfonyloxy, and propanesulfonyloxy groups. Preferably, these substituents on the aryl moiety are a halogen atom, particularly preferably a chlorine atom.

Preferable examples of a pyridyl-C₁₋₈ alkyl group represented by R include pyridyl-C₁₋₄ alkyl groups, concretely including pyridylmethyl, pyridylethyl, and pyridylpropyl groups. More preferable examples of a C₁₋₈ alkoxycarbonyl-C₁₋₈ alkyl group include C₁₋₄ alkoxycarbonyl-C₁₋₄ alkyl groups, concretely including methoxycarbonylmethyl, ethoxycarbonylmethyl, propoxycarbonylmethyl, methoxycarbonylethyl, and ethoxycarbonylethyl groups. Preferable examples of a carboxy-C₁₋₈ alkyl group include carboxy-C₁₋₄ alkyl groups, concretely including carboxymethyl and carboxyethyl groups.

A salt of the benzoic acid derivative includes: alkali metal salts such as sodium salts and potassium salts; alkaline-earthmetal salts such as calcium salts and magnesium salts; organic basic salts such as ammonium salts and trialkylamine salts; salts of mineral acids such as hydrochloride and sulfate; and salts of organic acids such as acetate.
The benzoic acid derivative of the present invention may be a solvate typified by a hydrate.

It is preferred that the benzoic acid derivative or the salt thereof of the present invention should be represented by the general formula (1), wherein A represents an oxygen atom, more preferably wherein X represents an oxygen atom or a sulfur atom, still more preferably wherein R represents a C₁₋₈ alkyl group or a C₆₋₁₀ aryl-oxy-C₁₋₈ alkyl group. In light of PPARα selectivity, a compound selected from 3-[3-[N-(Benzoxazol-2-yl)-N-3-(4-chlorophenoxy) ethyl]aminopropoxy]benzoic acid, 2-[5-[N-(Benzoxazol-2-yl)-N-isopropyl]aminopentylthio]benzoic acid, 2-[5-[N-(Benzoxazol-2-yl)-N-n-butyl]aminopentylthio]benzoic acid, 2-[5-[N-(Benzoxazol-2-yl)-N-n-pentyl]aminopentylthio]benzoic acid, and salts thereof is particularly preferred.

The compound of the present invention can be obtained by, for example, manufacturing methods shown by reaction schemes A and B described below.

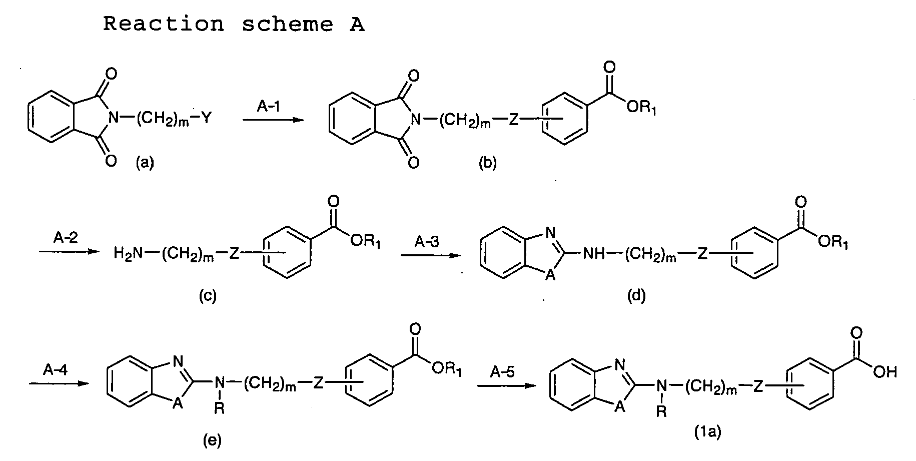

(in the formula, A, R, and m represent the same as above; R₁ represents a C₁₋₈ alkyl group; Y represents a halogen atom; and Z represents an oxygen atom, a sulfur atom, or an NH group.)

The manufacturing method shown by the reaction scheme A yields a compound (1a) of the present invention in the following steps: a monohalide (a) is obtained by the method described in Chemical and Pharmaceutical Bulletin (30 (5), pp 1579-1587, 1982) and then reacted with hydroxybenzoate or mercaptobenzoate; the resulting phthalimide (b) is treated with hydrazine to give an amine (c); this amine (c) is reacted with2-halo-benzoazoletogiveabenzoazole (d) ; thebenzoazole (d) is then reacted with any alkyl halide to give an ester (e) ; and the ester (e) is hydrolyzed to give the compound (1a).

The first step (A-1) is achieved by dissolving the monohalide (a) in a solvent such as DMF, THF, dioxane, or acetonitrile, which is then treated with required amounts of an inorganic base such as potassium carbonate (K₂CO₃), sodium carbonate (Na₂CO₃), or cesium carbonate (Cs₂CO₃) and an organic base such as triethylamine or diisopropylethylamine and subsequently with a required amount of hydroxybenzoate or mercaptobenzoate, and then heated at room temperature to a temperature around the boiling point of the solvent with stirring for several hours to 24 hours. The ester is appropriately selected from tert-butyl ester, ethyl ester, methyl ester, and the like.

The second step (A-2) is achieved by dissolving the phthalimide (b) of the raw material in a solvent such as methanol, ethanol, or n-propanol, which is then reacted with a required amount of hydrazine and then heated at room temperature to a temperature around the boiling point of the solvent with stirring for several hours to 24 hours.

The third step (A-3) is achieved by dissolving the amine (c) of the raw material in a solvent such as DMF, THF, dioxane, or acetonitrile, which is then added with 2-halo-1,3-benzoazole such as 2-chlorobenzoxazole in the presence of required amounts of an inorganic base such as K₂CO₃, Na₂CO₃, or CS₂CO₃ and an organic base such as triethylamine or diisopropylethylamine, and then stirred at room temperature to a temperature around the boiling point of the solvent for several hours to 24 hours, optionally under the atmosphere of inactive gas.

The fourth step (A-4) is achieved by dissolving the benzoazole (d) in a solvent such as DMF, THF, dioxane, or acetonitrile, which is then stirred together with any alkyl halide at room temperature to a temperature around the boiling point of the solvent in the presence of required amounts of an inorganic base such as K₂CO₃, Na₂CO₃, or CS₂CO₃ and an organic base such as triethylamine or diisopropylethylamine for several hours to 24 hours.

When the resulting product is obtained in the form of ester such as methyl ester or ethyl ester, which is easily hydrolyzed by an alkali, the fifth step (A-5) is achieved by dissolving the ester (e) in a solvent such as methanol, ethanol, or THF, which is then treated with a base such as lithium hydroxide, sodium hydroxide, or potassium hydroxide, or an aqueous solution thereof, and reacted for several hours to 24 hours under cooling or at room temperature to a temperature around the boiling point of the solvent, followed by acidification with an acid such as hydrochloric acid. Alternatively, when the resulting product is obtained in the form of ester such as tert-butyl ester, which is easily decomposed by an acid, the fifth step (A-5) is achieved by dissolving the ester (e) in a solvent such as dichloromethane or chloroform, which is then treated with an acid such as trifluoroacetic acid and then stirred for several hours to 24 hours under cooling or at room temperature.

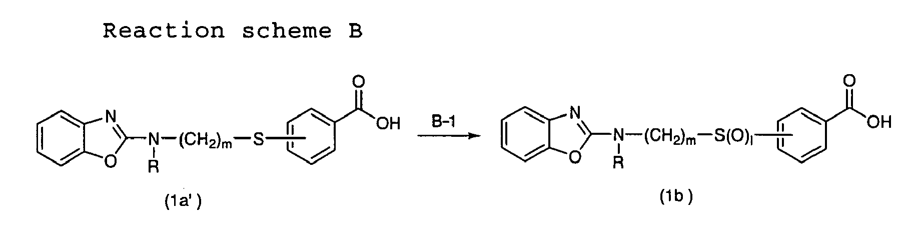

(in the formula, R and m represent the same as above; and 1 represents an integer of 1 or 2.)

The manufacturing method shown by the reaction scheme B yields sulfoxy and sulfone forms (1b) from a compound (1a') of the present invention obtained in the reaction step A-5.

In the first step (B-1), the compound (1a') of the present invention is dissolved in a solvent such as chloroform or dichloromethane and then oxidized with a peroxide such as m-chloroperoxybenzoic acid or H₂O₂. The reaction is achieved by stirring for several hours to 24 hours under cooling or at room temperature.

The compound of the present invention is obtained by the methods described above and optionally, can be purified using usual purification means such as recrystallization or column chromatography. The compound can optionally be converted to a desired salt or solvate described above by a routine method.

The compound of the present invention thus obtained has the effect of selectively activating PPARα as shown in Test Example below and as such, is useful as a preventive or therapeutic agent for hyperlipidemia, arteriosclerosis, diabetes, diabetes complications (e.g., diabetic nephropathy), inflammation, cardiac diseases, and so on, in mammals including humans, without weight gains and obesity.

A medicament of the present invention comprises the compound (1) of the present invention or the salt thereof as an active ingredient and can be used in a dosage form appropriately selected without particular limitations according to therapeutic purposes, which may be any of oral solid preparations, oral liquid preparations, injections, suppositories, topical agents, eye drops, nasal drops, ear drops, and adhesive preparations. The medicament in any of these dosage forms can be manufactured by a common preparation method known by those skilled in the art by mixing the compound (1) of the present invention with a pharmaceutically acceptable carrier.

When the oral solid preparation is prepared, the compound (1) of the present invention can be supplemented with an excipient and optionally with a binder, a disintegrant, a lubricant, a coloring agent, a flavoring agent, an odor-improving agent, and so on, and then processed into a tablet, agranule, apowder, a capsule, or the like, by a routine method. Such additives may be those generally used in the art and can be exemplified by: milk sugar, sodium chloride, grape sugar, starch, microcrystalline cellulose, and silicic acid as the excipient; water, ethanol, propanol, simple syrup, gelatin solutions, hydroxypropylcellulose, methylcellulose, ethylcellulose, shellac, calcium phosphate, and polyvinylpyrrolidone as the binder; agar powder, sodium hydrogencarbonate, sodium lauryl sulfate, and stearic acid monoglyceride as the disintegrant; purified talc, stearate, borax, and polyethylene glycol as the lubricant; β-carotene, yellow iron sesquioxide, and caramel as the coloring agent; and white sugar and orange peels as the flavoring agent.

When the oral liquid preparation is prepared, the compound (1) of the present invention can be supplemented with a flavoring agent, a buffer, a stabilizer, a preservative, and so on, to manufacture an oral liquid medicine, a syrup, an elixir, or the like by a routine method. Such additives may be those generally used in the art and include white sugar as the flavoring agent, sodium citrate as the buffer, traganth as the stabilizer, and p-hydroxybenzoic acid ester as the preservative.

When the injection is prepared, the compound (1) of the present invention can be supplemented with a pH regulator, a stabilizer, a tonicity agent, and so on, to manufacture hypodermic, intramuscular, and intravenous injections by a routine method. Such additives may be those generally used in the art and can be exemplified by sodium phosphate as the pH regulator, sodium pyrosulfite as the stabilizer, and sodium chloride as the tonicity agent.

When the suppository is prepared, the compound (1) of the present invention can be supplemented with a carrier and a surfactant to manufacture the suppository by a routine method. Such additives may be those generally used in the art and can be exemplified by polyethylene glycol and hard fat as the carrier and polysorbate 80 as the surfactant.

When the topical agent is prepared, the compound (1) of the present invention can be supplemented with a base, a water-solublepolymer, a solvent, a surfactant, apreservative, and so on, to manufacture a liquid medicine, a cream medicine, a gel medicine, an ointment, or the like, by a routine method. Such additives include: liquid paraffin, white Vaseline, and purified lanolin as the base; carboxyvinyl polymer as the water-soluble polymer; glycerin and water as the solvent; polyoxyethylene fatty acid ester as the surfactant; and p-hydroxybenzoic acid ester as the preservative.

When the eye drop is prepared, the compound (1) of the present invention can be supplemented with a pH regulator, a stabilizer, a tonicity agent, a preservative, and so on, to manufacture the eye drop by a routine method. Such additives may be those generally used in the art and can be exemplified by sodium phosphate as the pH regulator, sodium pyrosulfite and EDTA as the stabilizer, sodium chloride as the tonicity agent, and chlorobutanol as the preservative.

When the nasal drop is prepared, the compound (1) of the present invention can be supplemented with a pH regulator, a stabilizer, a tonicity agent, a preservative, and so on, to manufacture the nasal drop by a routine method. Such additives may be those generally used in the art and can be exemplified by sodium phosphate as the pH regulator, sodium pyrosulfite and EDTA as the stabilizer, sodium chloride as the tonicity agent, and benzalkonium chloride, as the preservative.

When the ear drop is prepared, the compound (1) of the present invention can be supplemented with a pH regulator, a buffer, a stabilizer, a tonicity agent, a preservative, and so on, to manufacture the ear drop by a routine method. Such additives may be those generally used in the art and can be exemplified by sodium phosphate as the pH regulator and the buffer, sodium pyrosulfite and EDTA as the stabilizer, sodium chloride as the tonicity agent, and benzalkonium chloride as the preservative.

When the adhesive preparation is prepared, the compound (1) of the present invention can be supplemented with an adhesive, a solvent, a cross-linking agent, a surfactant, and so on, to manufacture a wet adhesive preparation, an adhesive plaster, or the like, by a routine method. Such additives may be those generally used in the art and can be exemplified by: partially neutralized polyacrylic acid, sodium polyacrylate, 2-ethylhexyl polyacrylate, and styrene-isoprene-styrene block copolymers as the adhesive; glycerin and water as the solvent; dihydroxy aluminum aminoacetate and dried aluminum hydroxide gel as the cross-linking agent; and polyoxyethylene fatty acid ester as the surfactant.

The dose of the medicament of the present invention varies depending on, for example, ages, body weights, dosage forms, and the number of doses. In general, it is preferred that the medicament should be administered orally or parenterally to an adult at one dose or in several separate doses, of 1 to 1000 mg per day in terms of the amount of the compound (1) of the present invention.

### Example

Hereinafter, the present invention will be described with reference to Examples. However, the present invention is not intended to be limited to these Examples.

### Production Example 1

### Synthesis of methyl 3-(4-phthalimidobutoxy)benzoate

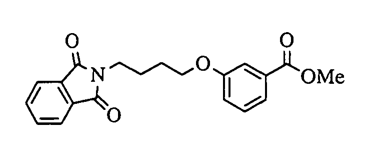

Methyl 3-hydroxybenzoate (3.0g, 19.7 mmol) was dissolved in 50 mL of acetonitrile and K₂CO₃ (3.27 g, 23.7 mmol) was added. The resulting mixture was reacted with N- (4-bromobutyl) phthalimide (6.68 g, 23.7 mmol) and KI (3.93 g, 23.7 mmol) and stirred overnight at 70°C. Following the addition of water, the resulting mixture was extracted with chloroform. The organic layer was washed with brine. The organic layer was dried with sodium sulfate and then concentrated under reduced pressure, followed by recrystallization with n-hexane/ethyl acetate to give a white crystal (6.60 g, 94.8%).

¹H-NMR(400MHz, CDCl₃) δ:
1.79-1.91(m, 4H), 3.75 (t, J = 7 Hz, 2H), 3.88 (s, 3H), 4.01 (t, J = 6 Hz, 2H), 7.05 (ddd, J = 8, 3, 1 Hz, 1H), 7.29 (t, J = 8 Hz, 1H), 7.49 (dd, J = 3, 2 Hz, 1H), 7.58 (dt, J = 8, 1 Hz, 1H), 7.69 (d, J = 3Hz, 1H), 7.70 (d, J = 3Hz, 1H), 7.82 (d, J = 3Hz, 1H), 7.84(d, J = 3Hz, 1H).

### Production Example 2

### Synthesis of methyl 3-(4-aminobutoxy)benzoate

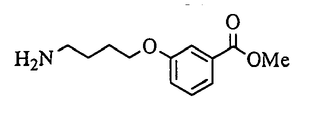

Methyl 3-(4-phthalimidobutoxy)benzoate (6.60 g, 18.68 mmol) was dissolved in 50 mL of ethanol and reacted with hydrazine monohydrate (1.87 g, 37.36 mmol). Following stirring at 80°C for 1 hour, the resulting mixture was diluted with chloroform, then washed with a saturated sodium bicarbonate solution, water, and brine, and dried with sodium sulfate. The resulting reaction solution was concentrated under reduced pressure to give a white crystal (4.81 g). The white crystal was directly used in subsequent reaction.

Production Example 3

### Synthesis of methyl 3-[4-(N-Benzoxazol-2-yl)aminobutoxy]benzoate

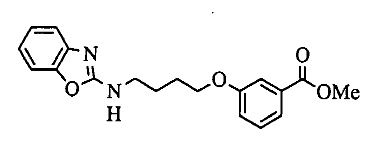

Methyl 3- (4-aminobutoxy)benzoate (4.81 g) was dissolved in 50.0 mL of tetrahydrofuran and N,N-diisopropylethylamine (2.90 g, 22.4 mmol) was added. Under cooling, 2-chlorobenzoxazole (3.44 g, 22.4 mmol) was added dropwise thereto and stirred overnight at room temperature. Following the addition of water, the resulting mixture was extracted with ethyl acetate. The organic layer was washed with brine. The organic layer was dried with sodium sulfate and then concentrated under reduced pressure, followed by purification by silica gel column chromatography (hexane:ethyl acetate = 4:1) to give a compound of interest (3.22 g, 50.7%).

¹H-NMR(400MHz, CDCl₃) δ:
1.66-1.75(m, 2H), 1.85-1.95(m, 2H), 3.54-3.61(br, 2H), 3.91(s, 3H), 4.06(t, J = 6Hz, 2H), 5.23(bs, 1H), 7.03(t, J = 8Hz, 1H), 7.07-7.10(m, 1H), 7.16(t, J = 8Hz, 1H), 7.23(d, J = 8Hz, 1H), 7.31-7.37(m, 2H), 7.55(s, 1H), 7.63(d, J = 8Hz, 1H)

Production Example 4

### Synthesis of methyl 3-[4-(N-Benzoxazol-2-yl)-N-n-propyl]aminobutoxy]benzoate

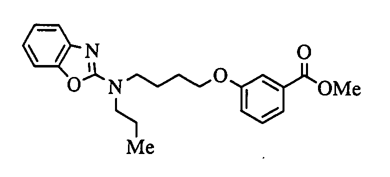

Methyl 3-[4-(N-Benzoxazol-2-yl)aminobutoxy]benzoate (4.0 g, 11.8 mmol) was dissolved in 5.0 mL of acetonitrile and cesium carbonate (4.97 g, 13.3 mmol) was added. Propyl iodide (2.59 g, 15.3 mmol) was then added dropwise to the reaction mixture. Following stirring overnight at 80°C, the resulting mixture was diluted with water and then extracted with ethyl acetate. The organic layer was washed with brine. The organic layer was dried with sodium sulfate and then concentrated under reduced pressure, followed by purification by silica gel column chromatography (hexane:ethyl acetate = 7:1) to give a titled compound (3.3 g, 73%).

¹H-NMR (400MHz, CDCl₃) δ:
0.97(t, J = 7Hz, 3H), 1.68-1.77(m, 2H), 1.84-1.95(m, 4H), 3.50(t, J = 8Hz, 2H), 3.58-3.67(br, 2H), 3.91(s, 3H), 4.02-4.12(br, 2H), 6.98(t, J = 7Hz, 1H), 7.07-7.16(m, 2H), 7.22(d, J = 8Hz, 1H), 7.31-7.37(m, 2H), 7.55(s, 1H), 7.62(d, J = 8Hz, 1H)

### Example 1

### Synthesis of 3-[4-(N-(Benzoxazol-2-yl)-N-n-propyl)aminobutoxy]benzoic acid

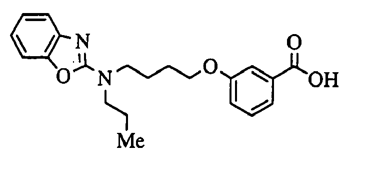

Methyl 3-[4-(N-Benzoxazol-2-yl)-N-n-propyl]aminobutoxy]benzoate (3.3 g, 8.57 mmol) was dissolved in 5.0 mL of methanol and 4.2 mL of 4.0 mol/L sodium hydroxide solution was added. After stirring at 80°C for 3 hours, the resulting mixture was concentrated under reduced pressure. After addition of diluted hydrochloric acid, the reaction mixture was extracted with chloroform. The organic layer was washed with brine. The organic layer was dried with sodium sulfate and then concentrated under reduced pressure,followed by purification by silica gel column chromatography (chloroform:methanol = 50:1) to give a titled compound (2.6 g, 82%).

¹H-NMR(400MHz, CDCl₃) δ:
0.98(t, J = 7Hz, 3H), 1.68-1.80(m, 2H), 1.84-2.00(br, 4H), 3.51(t, J = 8Hz, 2H), 3.63(t, 7Hz, 2H), 4.05(t, J = 6Hz, 2H), 6.99(t, J = 8Hz, 1H), 7.09-7.17(m, 2H), 7.23(d, J = 8Hz, 1H), 7.34(t, J = 8Hz, 1H), 7.40(d, J = 8Hz, 1H), 7.60(s, 1H), 7.70(d, J = 8Hz, 1H)
Hereinafter, compounds of Examples 2 to 111 were synthesized in the same way as in Example 1.

### Example 2

### 3-[4-(N-(Benzoxazol-2-yl)-N-methyl)aminobutoxy]benzoic acid

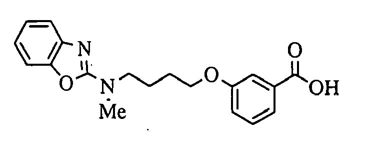

¹H-NMR (270MHz, CDCl₃) δ:
1.79-1.99 (m, 4H), 3.23 (s, 3H), 3.56-3.73 (m, 2H), 3.98-4.14 (m, 2H), 7.00 (t, 1H, J = 8H), 7.06-7.29 (m, 3H), 7.33 (d, J = 9Hz, 1H), 7.38 (t, J = 9Hz, 1H), 7.59 (s, 1H), 7.69 (d, J = 8Hz, 1H).

### Example 3

### 3-[4-(N-(Benzoxazol-2-yl)-N-ethyl)aminobutoxy]benzoic acid

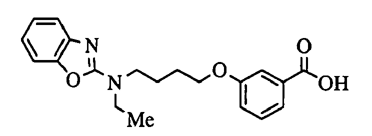

¹H-NMR (400MHz, CDCl₃) δ:
1.30 (t, J = 7 Hz, 3H), 1.81-1.98 (m, 4H), 3.56-3.68 (m, 4H), 4.05 (t, J = 6 Hz, 2H), 6.99 (dt, J = 8, 1 Hz, 1H), 7.10 (dd, J = 8, 2 Hz, 1H), 7.15 (t, J = 8 Hz, 1H), 7.24 (d, J = 8 Hz, 1H), 7.34 (t, J = 8 Hz, 1H), 7.41 (dd, J = 8, 1 Hz, 1H), 7.61 (s, 1H), 7.70 (d, J = 8 Hz, 1H).

Example 4

### 3-[4-(N-(Benzoxazol-2-yl)-N-n-butyl)aminobutoxy]benzoic acid

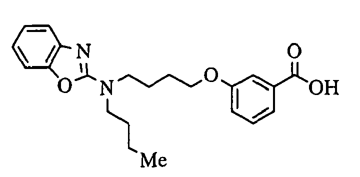

¹H-NMR (270MHz, CDCl₃) δ:
0.97 (t, J = 7 Hz, 3H), 1.40 (sextet, J = 7 Hz, 2H), 1.69 (quintet, J = 7 Hz, 2H), 1.78-1.97 (m, 4H), 3.54 (t, J = 7 Hz, 2H), 3.60-3.62 (m, 2H), 3.97-4.09 (m, 2H), 6.99 (t, J = 8 Hz, 1H), 7.08 (dd, J = 8, 2 Hz, 1H), 7.15 (t, J = 8 Hz, 1H), 7.24 (d, J = 9 Hz, 1H), 7.32 (t, J = 8 Hz, 1H), 7.42 (d, J = 7 Hz, 1H), 7.56 (s, 1H), 7.70 (d, J = 8 Hz, 1H).

Example 5

### 3-[3-[N-(Benzoxazol-2-yl)-N-3-phenoxypropyl]aminopropoxy]benzoic acid

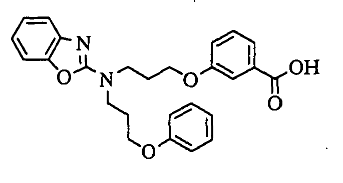

¹H-NMR (400MHz, CD₃OD) δ:
2.20 (m, 4H), 3.78 (m, 4H), 4.05 (t, J = 6 Hz, 2H), 4.10 (t, J = 6 Hz, 2H), 6.85-6.87 (m, 3H), 6.95 (td, J = 8, 1 Hz, 1H), 7.01 (dd, J = 7, 2 Hz, 1H), 7.09-7.19 (m, 2H), 7.19-7.26 (m, 4H), 7.52-7.53 (m, 2H).

### Example 6

### 3-[3-[N-(Benzoxazol-2-yl)-N-3-(4-chlorophenoxy) ethyl]aminopropoxy]benzoic acid

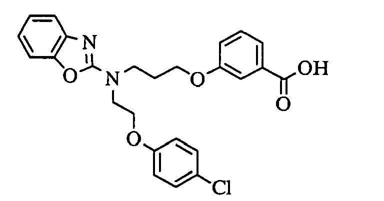

¹H-NMR (DMSO-d₆) δ:
2.16 (t, J = 7Hz, 2H), 3.79 (t, J = 7Hz, 2H), 3.91 (t, J = 6Hz, 2H), 4.08 (t, J = 6 Hz, 2H), 4.26 (t, J = 6 Hz, 2H), 6.95-7.00 (m, 4H), 7.19 (td, J = 8, 1 Hz, 1H), 7.24 (t, J = 8 Hz, 1H), 7.29-7.34 (m, 4H), 7.59 (dd, J = 8, 2 Hz, 2H).

### Example 7

### 2-[2-[N-(Benzoxazol-2-yl)-N-1-naphthylmethyl]aminoethoxy]benzoic acid

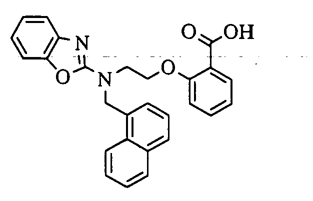

MS (m/z) 438 (M⁺)

### Example 8

### 2-[2-[N-(Benzoxazol-2-yl)-N-2-nitrobenzyl]aminoethoxy]benzoic acid

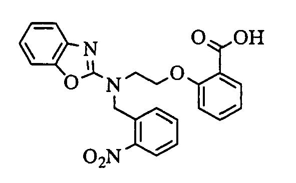

MS (m/z) 433 (M⁺)

### Example 9

### 2-[2-[N-(Benzoxazol-2-yl)-N-4-chlorobenzyl]aminoethoxy]benzoic acid

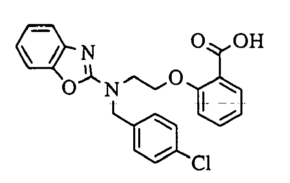

MS (m/z) 422 (M⁺), 424 (M⁺+2)

### Example 10

### 2-[2-[N-(Benzoxazol-2-yl)-N-4-fluorobenzyl]aminoethoxy]benzoic acid

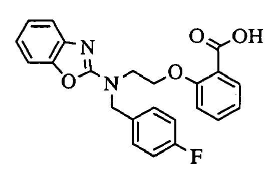

MS (m/z) 406 (M⁺)

### Example 11

### 2-[2-(N-(Benzoxazol-2-yl) -N-n-butyl]aminoethoxy]benzoic acid

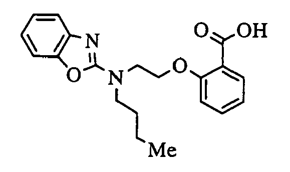

MS (m/z) 354 (M⁺)

### Example 12

### 2-[2-[N-(Benzoxazol-2-yl)-N-n-heptyl]aminoethoxy]benzoic acid

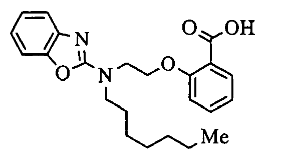

¹H-NMR (270MHz, CDCl₃) δ:
0.85 (t, J = 7 Hz, 3H), 1.21-1.33 (m, 8H), 1.70 (t, J = 7Hz, 2H), 3.64 (t, J = 8Hz, 2H), 4.04 (t, J = 5Hz, 2H), 4.48 (t, J = 5Hz, 2H), 6.94-7.13 (m, 3H), 7.16 (t, J = 8Hz, 1H), 7.27 (d, J = 7Hz, 1H), 7.40 (d, J = 7Hz, 1H), 7.50 (t, J = 7Hz, 1H), 8.09 (dd, J = 8, 2 Hz, 1H).

### Example 13

### 2-[2-[N-(Benzoxazol-2-yl)-N-n-octyl]aminoethoxy]benzoic acid

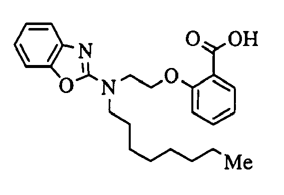

MS (m/z) 410 (M⁺)

### Example 14

### 2-[2-[N-(Benzoxazol-2-yl)-N-n-propyl]aminoethoxy]benzoic acid

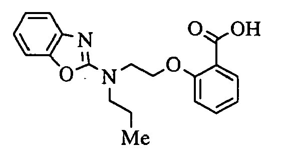

¹H-NMR (270MHz, CDCl₃) δ:
0.97 (t, J = 8 Hz, 3H), 1.67-1.81 (m, 2H), 3.62 (t, J = 8Hz, 2H), 4.04 (t, J=5Hz, 2H), 4.47 (t, J= 5Hz, 2H), 6.91-7.10 (m, 3H), 7.16 (t, J = 7Hz, 1H), 7.27 (d, J = 8Hz, 1H), 7.40 (d, J = 8Hz, 1H), 7.47 (t, J = 7Hz, 1H), 8.08 (dd, J = 8, 2 Hz, 1H) .

### Example 15

### 3-[3-[N-(Benzoxazol-2-yl)-N-4-chlorobenzyl]aminopropoxy]benzoic acid

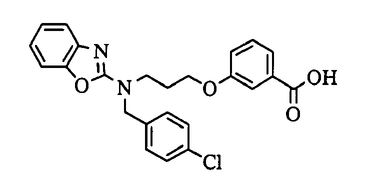

MS (m/z) 436 (M⁺), 438 (M⁺+2)

### Example 16

### 3-[3-[N-(Benzoxazol-2-yl)-N-ethyl]aminopropoxy]benzoic acid

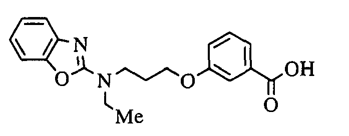

MS (m/z) 340 (M⁺)

### Example 17

### 3-[3-[N-(Benzoxazol-2-yl)-N-methyl]aminopropoxy]benzoic acid

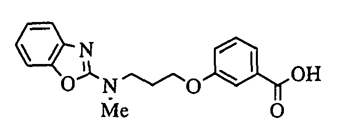

MS (m/z) 326 (M⁺)

### Example 18

### 3- [3- [N- (Benzoxazol-2-yl) -N-n-butyl]aminopropoxy]benzoic acid

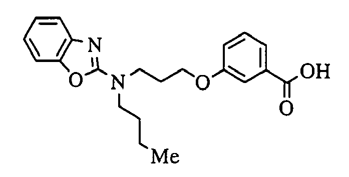

MS (m/z) 368 (M⁺)

### Example 19

### 3-[3-[N-(Benzoxazol-2-yl)-N-n-heptyl]aminopropoxy]benzoic acid

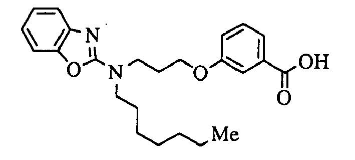

MS (m/z) 410 (M⁺)

### Example 20

### 3- [3- [N- (Benzoxazol-2-yl) -N-n-octyl]aminopropoxy]benzoic acid

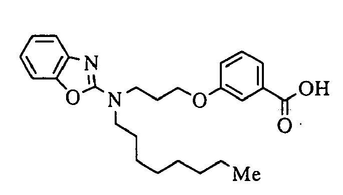

MS (m/z) 424 (M⁺)

### Example 21

### 3-[3-[N-(Benzoxazol-2-yl)-N-n-propyl]aminopropoxy]benzoic acid

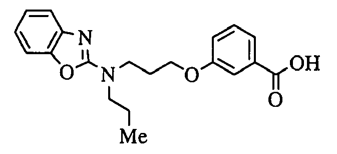

MS (m/z) 354 (M⁺)

### Example 22

### 3-[3-[N-(Benzoxazol-2-yl)-N-3-phenylpropyl]aminopropoxy]benzoic acid

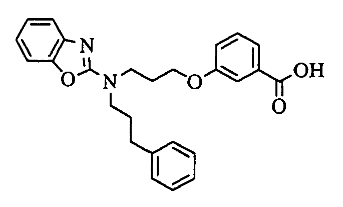

MS (m/z) 430 (M⁺)

### Example 23

### 2-[3-[N-(Benzoxazol-2-yl)-N-4-chlorobenzyl]aminopropoxy]benzoic acid

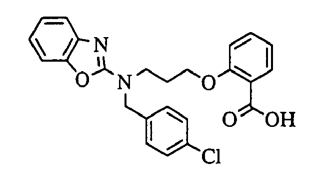

MS (m/z) 436 (M⁺), 438 (M⁺+2)

### Example 24

### 2-[3-[N-(Benzoxazol-2-yl)-N-4-fluorobenzyl]aminopropoxy]benzoic acid

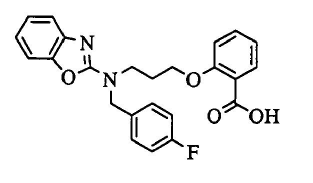

MS (m/z) 420 (M⁺)

### Example 25

### 2-[3-[N-(Benzoxazol-2-yl)-N-n-butyl]aminopropoxy]benzoic acid

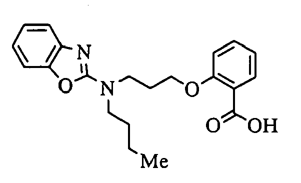

MS (m/z) 368 (M⁺)

### Example 26

### 2-[3-[N-(Benzoxazol-2-yl)-N-n-hexyl]aminopropoxy]benzoic acid

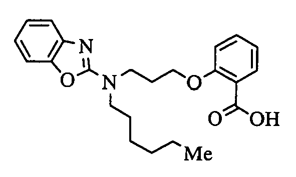

MS (m/z) 396 (M⁺)

### Example 27

### 2-[3-[N-(Benzoxazol-2-yl)-N-n-octyl]aminopropoxy]benzoic acid

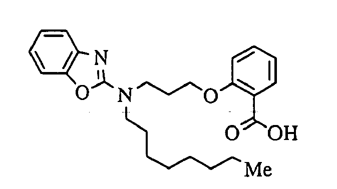

MS (m/z) 424 (M⁺)

### Example 28

### 2-[3-[N-(Benzoxazol-2-yl)-N-n-propyl]aminopropoxy]benzoic acid

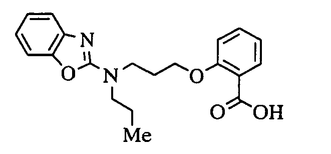

MS (m/z) 354 (M⁺)

### Example 29

### 2-[3-[N-(Benzoxazol-2-yl)-N-3-phenylpropyl]aminopropoxy]benzoic acid

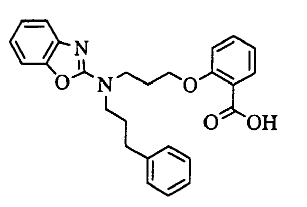

MS (m/z) 430 (M⁺)

### Example 30

### 2-[3-[N-(Benzoxazol-2-yl)-N-2-phenoxyethyl]aminopropoxy]benzoic acid

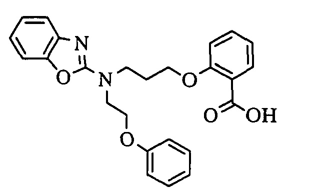

MS (m/z) 432 (M⁺)

### Example 31;

### 2-[3-[N-(Benzoxazol-2-yl)-N-3-phenoxypropyl]aminopropoxy]benzoic acid

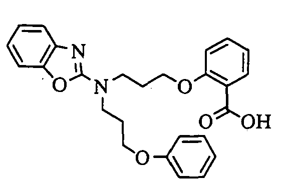

MS (m/z) 446 (M⁺)

### Example 32

4-[3-[N-(Benzoxazol-2-yl)-N-4-chorobenzyl]aminopropoxy]benzoic acid

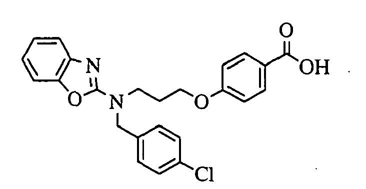

MS (m/z) 436 (M⁺), 438 (M⁺+2)

### Example 33

### 4- [3-[N-(Benzoxazol-2-yl)-N-2-(4-chorophenoxy)ethyl]aminopropoxy]benzoic acid

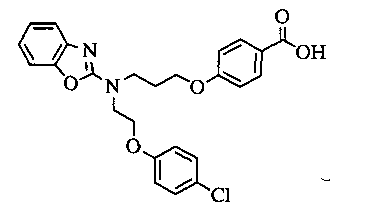

MS (m/z) 466 (M⁺), 468 (M⁺+2)

### Example 34

### 4-[3-[N-(Benzoxazol-2-yl)-N-ethyl]aminopropoxy]benzoic acid

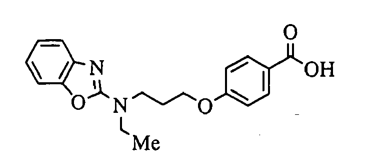

MS (m/z) 340 (M⁺)

### Example 35

### 4-[3-[N-(Benzoxazol-2-yl)-N-methyl]aminopropoxy]benzoic acid

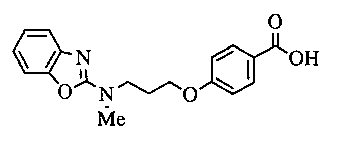

MS (m/z) 326 (M⁺)

### Example 36

### 4-[3-[N-(Benzoxazol-2-yl)-N-n-butyl]aminopropoxy]benzoic acid

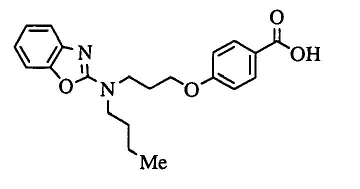

MS (m/z) 368 (M⁺)

### Example 37

### 4-[3-[N-(Benzoxazol-2-yl)-N-n-heptyl]aminopropoxy]benzoic acid

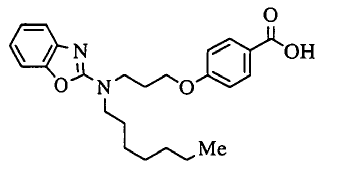

MS (m/z) 410 (M⁺)

### Example 38

### 4-[3-[N-(Benzoxazol-2-yl)-N-n-hexyl]aminopropoxy]benzoic acid

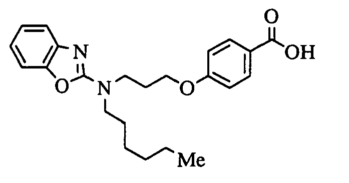

MS (m/z) 396 (M⁺)

### Example 39

### 4-[3-[N-(Benzoxazol-2-yl)-N-n-octyl] aminopropoxy]benzoic acid

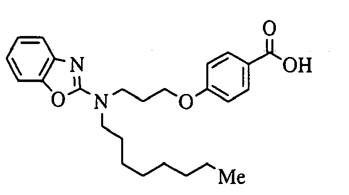

MS (m/z) 424 (M⁺)

### Example 40

### 4-[3-[N-(Benzoxazol-2-yl)-N-n-propyl]aminopropoxy]benzoic acid

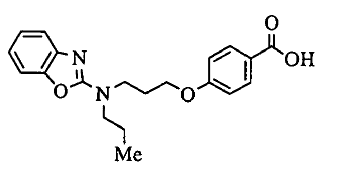

MS (m/z) 354 (M⁺)

### Example 41

### 4- [3- [N- (Benzoxazol-2-yl) -N-3-phenylpropyl]aminopropoxy]benzoic acid

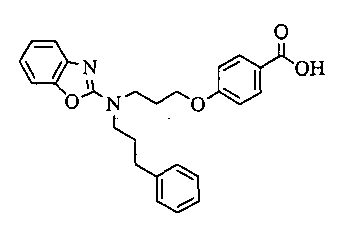

MS (m/z) 430 (M⁺)

### Example 42

### 4-[3-[N-(Benzoxazol-2-yl)-N-3-phenoxypropyl]aminopropoxy]benzoic acid

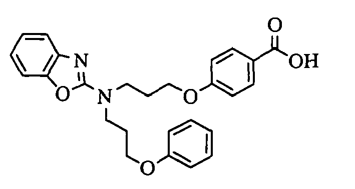

MS (m/z) 446 (M⁺)

### Example 43

### 3-(4-[N-(Benzoxazol-2-yl)-N-4-chlorobenzyl]aminobutoxy]benzoic acid

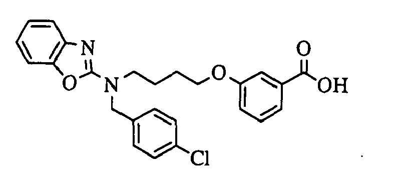

MS (m/z) 450 (M⁺), 452 (M⁺+2)

### Example 44

### 3-[4-[N-(Benzoxazol-2-yl)-N-3-phenylpropyl]aminobutoxy]benzoic acid

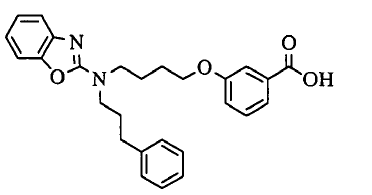

MS (m/z) 444 (M⁺),

### Example 45

### 3-[4-[N-(Benzoxazol-2-yl)-N-2-(4-chloropheoxyl)ethyl]aminobutoxy]benzoic acid

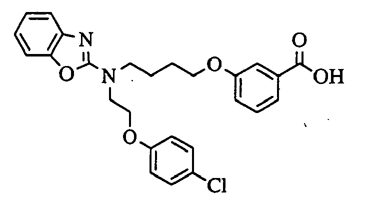

MS (m/z) 480 (M⁺), 482 (M⁺+2)

Example 46

### 3-[4-[N-(Benzoxazol-2-yl)-N-n-heptyl]aminobutoxy]benzoic acid

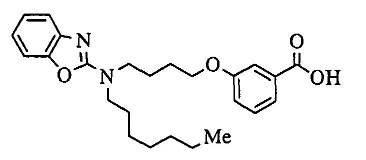

MS (m/z) 424 (M⁺)

### Example 47

### 3-[4-[N-(Benzoxazol-2-yl)-N-n-pentyl]aminobutoxy]benzoic acid

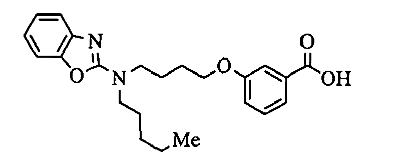

MS (m/z) 395 (M⁺)

### Example 48

### 3- [4- [N- (Benzoxazol-2-yl) -N-3-phenoxypropyl]aminobutoxy]benzoic acid

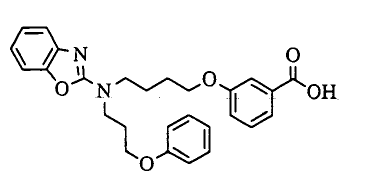

MS (m/z) 460 (M⁺)

### Example 49

### 2-[4-[N-(Benzoxazol-2-yl)-N-4-chlorobenzyl]aminobutoxy]benzoic acid

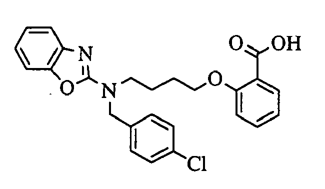

MS (m/z) 450 (M⁺), 452 (M⁺+2)

### Example 50

### 2-[4-[N-(Benzoxazol-2-yl)-N-n-octyl]aminobutoxy]benzoic acid

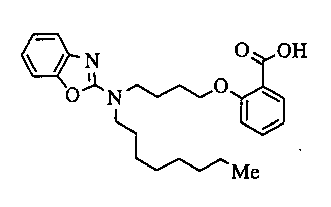

MS (m/z) 438 (M⁺)

### Example 51

### 2-[4-[N-(Benzoxazol-2-yl)-N-n-propyl]aminobutoxy]benzoic acid

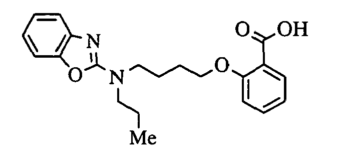

MS (m/z) 368 (M⁺)

### Example 52

### 2-[4-[N-(Benzoxazol-2-yl)-N-2-phenoxyethyl]aminobutoxy]benzoic acid

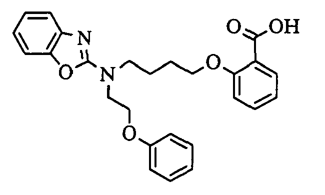

MS (m/z) 446 (M⁺)

### Example 53

### 2-[4-[N-(Benzoxazol-2-yl)-N-3-phenoxypropyl]aminobutoxy]benzoic acid

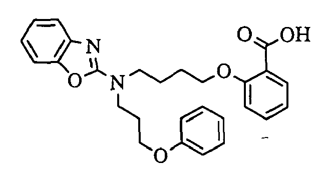

MS (m/z) 460 (M⁺)

### Example 54

### 4-[4-[N-(Benzoxazol-2-yl)-N-4-chlorobenzyl]aminobutoxy]benzoic acid

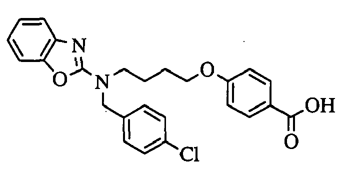

MS (m/z) 450 (M⁺), 452 (M⁺+2)

### Example 55

### 4-[4-[N-(Benzoxazol-2-yl)-N-2-(4-chlorophenoxy)ethyl]aminobutoxy]benzoic acid

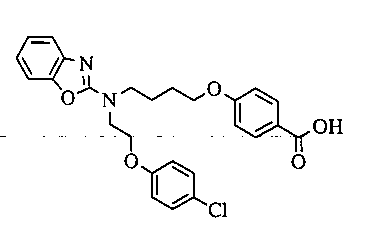

MS (m/z) 480 (M⁺), 482 (M⁺+2)

### Example 56

### 4-[4-[N-(Benzoxazol-2-yl)-N-ethyl]aminobutoxy]benzoic acid

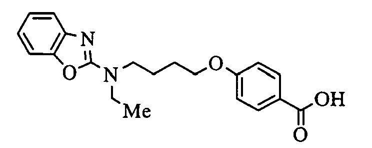

MS (m/z) 354 (M⁺)

### Example 57

### 4-[4-[N-(Benzoxazol-2-yl)-N-methyl]aminobutoxy]benzoic acid

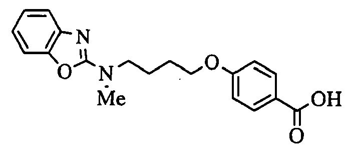

MS (m/z) 340 (M⁺)

### Example 58

### 4-[4-[N-(Benzoxazol-2-yl)-N-n-butyl]aminobutoxy]benzoic acid

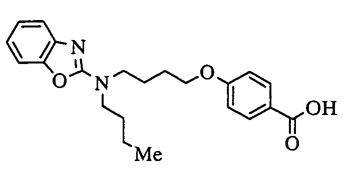

MS (m/z) 382 (M⁺)

### Example 59

### 4-[4-[N-(Benzoxazol-2-yl)-N-n-heptyl]aminobutoxy]benzoic acid

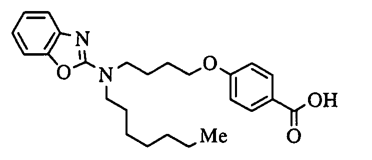

MS (m/z) 424 (M⁺)

### Example 60

### 4-[4-[N-(Benzoxazol-2-yl)-N-n-hexyl]aminobutoxy]benzoic acid

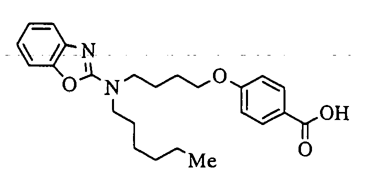

MS (m/z) 410 (M⁺)

### Example 61

### 4-[4-[N-(Benzoxazol-2-yl)-N-n-octyl]aminobutoxy]benzoic acid

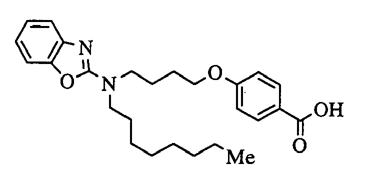

MS (m/z) 438 (M⁺)

### Example 62

### 4-[4-[N-(Benzoxazol-2-yl)-N-n-propyl]aminobutoxy]benzoic acid

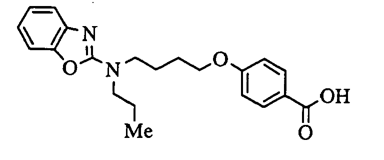

MS (m/z) 368 (M⁺)

### Example 63

### 4-[4-[N-(Benzoxazol-2-yl)-N-3-phenylpropyl]aminobutoxy]benzoic acid

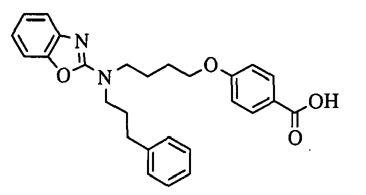

MS (m/z) 444 (M⁺)

Example 64

### 4-[4-[N-(Benzoxazol-2-yl)-N-3-phenoxypropyl]aminobutoxy]benzoic acid

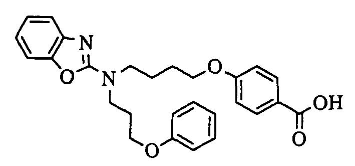

MS (m/z) 460 (M⁺)

### Example 65

### 2-[3-[N-(Benzoxazol-2-yl)-N-2-phenoxyethyl]aminopropylthio]benzoic acid

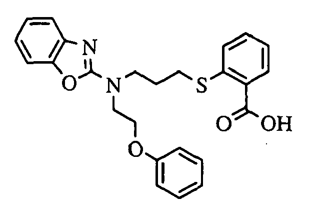

¹H-NMR (400MHz, CDCl₃) δ:
2.21 (quintet, J = 7Hz, 2H), 3.03 (t, J = 7Hz, 2H), 3.87 (t, J = 7Hz, 2H), 3.96 (t, J = 5Hz, 2H), 4.25 (t, J = 5Hz, 2H), 6.85 (d, J = 8Hz, 2H), 6.92 (t, J = 7Hz, 1H), 7.01 (t, J = 8Hz, 1H), 7.14-7.26 (m, 5H),7.30 (d, J = 7Hz, 1H), 7.37-7.43 (m, 2H), 8.07 (d, J = 8Hz, 1H)

### Example 66

### 2-[4-[N-(Benzoxazol-2-yl)-N-ethyl]aminobutylthio]benzoic acid

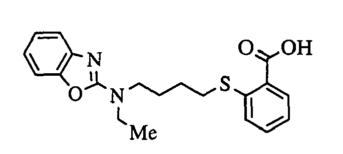

¹H-NMR (400MHz, DMSO-d₆) δ:
1.20 (t, J = 7 Hz, 3H), 1.66 (tt, J = 7, 7 Hz, 2H), 1.81 (tt, J = 7, 7 Hz, 2H), 2.99 (t, J = 7 Hz, 2H), 3.50-3.55 (m, 4H), 6.97 (t, J = 8 Hz, 1H), 7.12 (t, J = 8 Hz, 1H), 7.19 (t, J = 7 Hz, 1H), 7.25 (d, J = 8 Hz, 1H), 7.36-7.47 (m, 3H), 7.84 (d, J = 8 Hz, 1H), 13.0 (s, 1H).

### Example 67

### 2-[4-[N-(Benzoxazol-2-yl)-N-methyl]aminobutylthio]benzoic acid

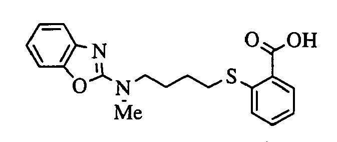

¹H NMR (400MHz, DMSO-d₆) δ:
1.65 (tt, J = 8, 7 Hz, 2H), 1.80 (tt, J = 8, 7 Hz, 2H), 2.98 (t, J = 7 Hz, 2H), 3.11 (s, 3H), 3.55 (t, J = 7 Hz, 2H), 6.97 (td, J = 8, 1 Hz, 1H), 7.12 (td, J = 8, 1 Hz, 1H), 7.18 (t, J = 8 Hz, 1H), 7.25 (d, J = 7 Hz, 1H), 7.38 (t, J = 8 Hz, 2H), 7.46 (td, J = 8, 2 Hz, 1H), 7.84 (d, J = 8 Hz, 1H), 13.0 (s, 1H).

### Example 68

### 2-[4-[N-(Benzoxazol-2-yl)-N-n-propyl]aminobutylthio]benzoic acid

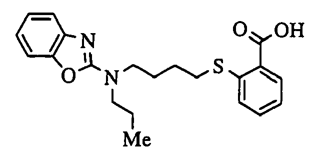

¹H-NMR (400MHz, CDCl₃) δ:
0.95 (t, J = 7Hz, 3H), 1.64-1.78 8 (m, 4H), 1.80-1.95 (m, 2H), 2.92 (t, J = 7Hz, 2H), 3.45 (t, J = 8Hz, 2H), 3.53 (t, J = 8Hz, 2H), 6.99 (t, J = 7Hz, 1H), 7.13-7.17 (m, 2H), 7.23 (d, J = 8Hz, 1H), 7.30-7.37 (m, 2H), 7.46 (d, J = 8Hz, 1H), 7.98 (d, J = 7Hz, 1H)

### Example 69

### 2-[5-[N-(Benzoxazol-2-yl)-N-cyclohexylmethyl]aminopentylthio]benzoic acid

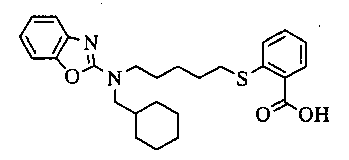

¹H-NMR (400MHz, CDCl₃) δ:
0.98-1.03 (m, 2H), 1.15-1.24 (m, 3H), 1.51-1.55 (m, 2H), 1.71-1.78 (m, 10H), 2.84 (t, J = 7 Hz, 2H), 3.35 (d, J = 7 Hz, 2H), 3.52 (t, J = 8 Hz, 2H), 6.99 (dt, J = 8, 1 Hz, 1H), 7.14 (dt, J = 8, 1 Hz, 1H), 7.19 (d, J = 8 Hz, 1H), 7.24 (d, J = 8 Hz, 1H), 7.28 (d, J = 8 Hz, 1H), 7.38-7.42 (m, 2H), 8.07 (dd, J = 8, 1 Hz, 1H).

### Example 70

### 2-[5-[N-(Benzoxazol-2-yl)-N-isopropyl]aminopentylthio]benzoic acid

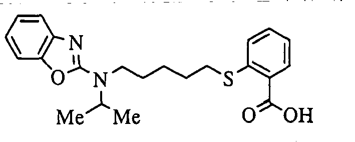

¹H-NMR (400MHz, CDCl₃) δ:
1.30 (d, J = 7 Hz, 6H), 1.54 (tt, J = 8, 7 Hz, 2H), 1.77 (quintet, J = 8 Hz, 4H), 2.86 (t, J = 7 Hz, 2H), 3.41 (t, J = 8 Hz, 2H), 4.47 (septet, J = 7 Hz, 1H), 6.99 (td, J = 8, 1 Hz, 1H), 7.14 (td, J = 8, 1 Hz, 1H), 7.19 (t, J = 8 Hz, 1H), 7.23-7.30 (m, 2H), 7.39-7.43 (m, 2H), 8.08 (dd, J = 8, 2 Hz, 1H).

### Example 71

### 2-[5-[N-(Benzoxazol-2-yl)-N-n-butyl]aminopentylthio] benzoic acid

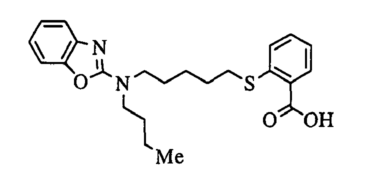

¹H-NMR (270MHz, CDCl₃) δ:
0.96 (t, J = 7Hz, 3H), 1.38-1.85 (m, 10H), 2.87 (t, J = 7Hz, 2H), 3.45-3.62 (m, 4H), 6.99 (t, J = 8Hz, 1H), 7.11-7.34 (m, 6H), 8.08 (dd, J = 8, 1Hz, 1H).

### Example 72

### 2-[5-[N-(Benzoxazol-2-yl)-N-n-pentyl]aminopentylthio]benzoic acid

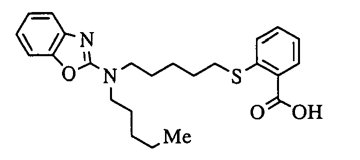

¹H-NMR (CDCl₃) δ:
0.89 (t, J = 7Hz, 3H), 1.25-1.48 (m, 8H), 1.64 (q, J = 7Hz, 4H), 2.80 (br, 2H), 3.45 (t, J = 7Hz, 4H), 6.96 (t, J = 8Hz, 1H), 7.11 (m, 1H), 7.12 (t, J = 8Hz, 1H), 7.21-7.26 (m, 2H), 7.31 (m, 1H), 7.36 (d, J = 8Hz, 1H), 7.99 (d, J = 8Hz, 1H).

### Example 73

### 2-[2-[N-(Benzoxazol-2-yl)-N-(2-methyl-3-nitrobenzyl)]aminoethylthio]benzoic acid

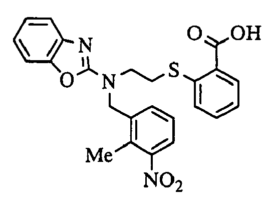

MS (m/z) 463 (M⁺)

### Example 74

### 2-[2-[N-(Benzoxazol-2-yl)-N-2-nitrobenzyl]aminoethylthio]benzoic acid

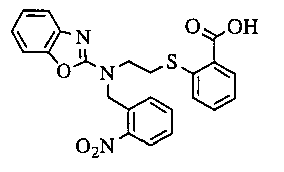

MS (m/z) 449 (M⁺)

### Example 75

### 2-[2-[N-(Benzoxazol-2-yl) N-4-chlorobenzyl]aminoethylthio]benzoic acid

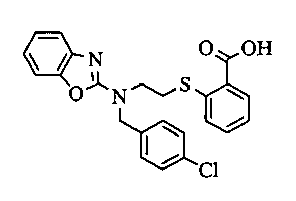

MS (m/z) 438 (M⁺), 440 (M⁺+2)

### Example 76

### 2-[2-[N-(Benzoxazol-2-yl)-N-n-butyl]aminoethylthio]benzoic acid

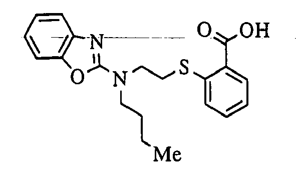

MS (m/z) 370 (M⁺)

### Example 77

### 2-[2-[N-(Henzoxazol-2-yl)-N-n-heptyl]aminoethylthio] benzoic acid

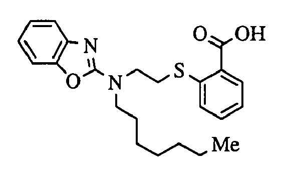

MS (m/z) 412 (M⁺)

### Example 78

### 2-[2-[N-(Benzoxazol-2-yl)-N-n-octyl]aminoethylthio]benzoic acid

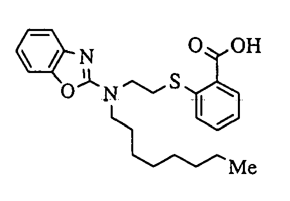

MS (m/z) 426 (M⁺)

### Example 79

### 2-[2-(N-(Benzoxazol-2-yl)-N-3-phenylpropyl]aminoethylthio]benzoic acid

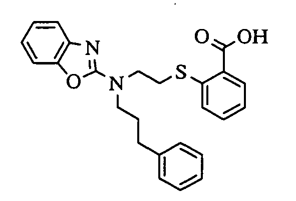

MS (m/z) 432 (M⁺)

### Example 80

### 2-[2-[N-(Benzoxazol-2-yl)-N-3-phenoxypropyl]aminoethylthio]benzoic acid

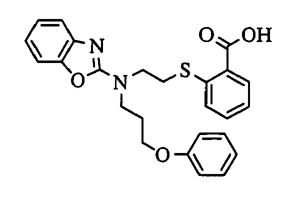

MS (m/z) 448 (M⁺)

### Example 81

### 2-[3-[N-(Benzoxazol-2-yl)-N-4-chlorobenzyl]aminopropylthio]benzoic acid

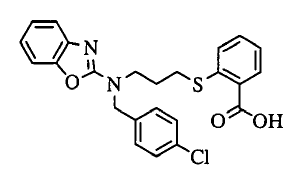

MS (m/z) 453 (M⁺), 455 (M⁺+2)

### Example 82

### 2-[3-[N-(Benzoxazol-2-yl)-N-ethyl]aminopropylthio]benzoic acid

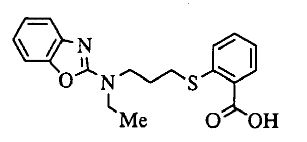

MS (m/z) 356 (M⁺)

### Example 83

### 2-[3-[N-(Benzoxazol-2-yl)-N-isopropyl]aminopropylthio]benzoic acid

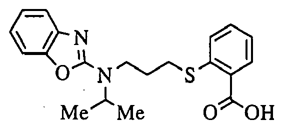

MS (m/z) 370 (M⁺)

### Example 84

### 2-[3-[N-(Benzoxazol-2-yl)-N-n-butyl]aminopropylthio]benzoic acid

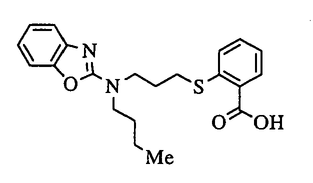

MS (m/z) 385 (M⁺)

.

### Example 85

### 2-[3-[N-(Benzoxazol-2-yl)-N-n-hexyl]aminopropylthio]benzoic acid

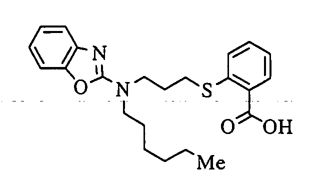

MS (m/z) 413 (M⁺)

### Example 86

### 2-[3-[N-(Benzoxazol-2-yl)-N-n-octyl]aminopropylthio]benzoic acid

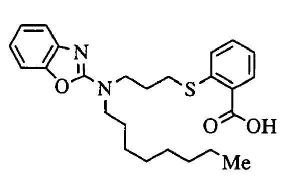

MS (m/z) 441 (M⁺)

### Example 87

### 2-[3-[N-(Benzoxazol-2-yl)-N-n-pentyl]aminopropylthio]benzoic acid

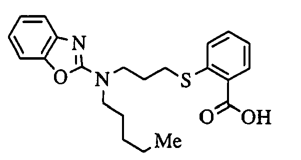

MS (m/z) 398 (M⁺)

### Example 88

### 2-[3-[N-(Benzoxazol-2-yl)-N-n-propyl]aminopropylthio]benzoic acid

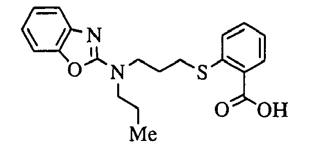

MS (m/z) 370 (M⁺)

### Example 89

### 2-[3-[N-(Benzoxazol-2-yl)-N-3-phenylpropyl]aminopropylthio]benzoic acid

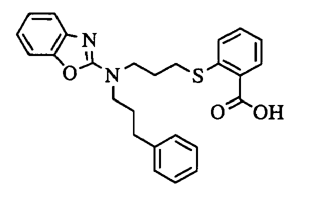

MS (m/z) 447 (M⁺)

### Example 90

2-[4-[N-(Benzoxazol-2-yl)-N-4-chlorobenzyl]aminobutylthio]benzoic acid

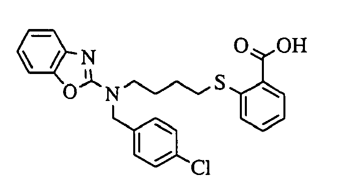

MS (m/z) 467 (M⁺), 469 (M⁺+2)

### Example 91

### 2-[4-[N-(Benzoxazol-2-yl)-N-cyclohexylmethyl]aminobutylthio]benzoic acid

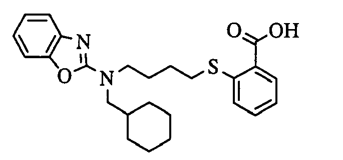

MS (m/z) 439 (M⁺)

### Example 92

### 2-[4-[N-(Benzoxazol-2-yl)-N-n-butyl]aminobutylthio]benzoic acid

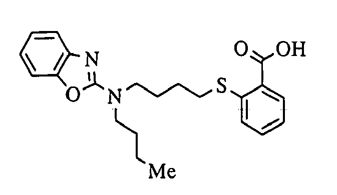

MS (m/z) 399 (M⁺)

### Example 93

### 2-[4-[N-(Benzoxazol-2-yl)-N-n-hexyl]aminobutylthio]benzoic acid

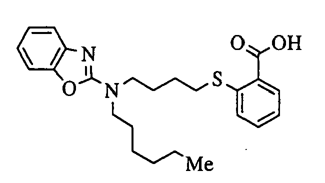

MS (m/z) 427 (M⁺)

### Example 94

### 2-[4-[N-(Benzoxazol-2-yl)-N-n-octyl]aminobutylthio]benzoic acid

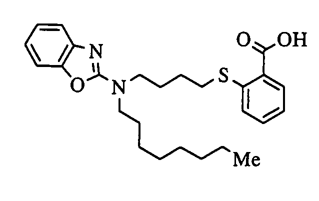

MS (m/z) 455 (M⁺)

### Example 95

### 2-[4-[N-(Benzoxazol-2-yl)-N-n-pentyl]aminobutylthio] benzoic acid

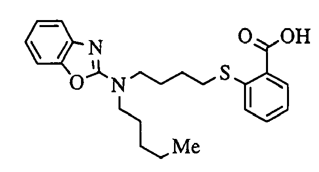

MS (m/z) 413 (M⁺)

### Example 96

### 2-[4-[N-(Benzoxazol-2-yl)-N-benzyl]aminobutylthio]benzoic acid

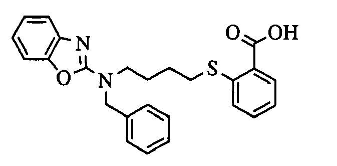

MS (m/z) 433 (M⁺)

### Example 97

### 2-[4-[N-(Benzoxazol-2-yl) N-3-phenylpropyl]aminobutylthio]benzoic acid

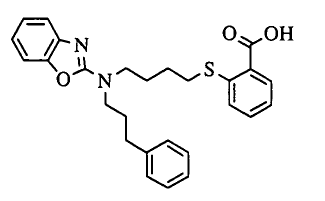

MS (m/z) 461 (M⁺)

### Example 98

### 2-[5-[N-(Benzoxazol-2-yl)-N-4-chlorobenzyl]aminopentylthio]benzoic acid

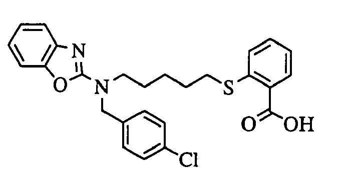

MS (m/z) 480 (M⁺), 482 (M⁺+2)

### Example 99

### 2-[5-[N-(Benzoxazol-2-yl)-N-ethyl]aminopentylthio]benzoic acid

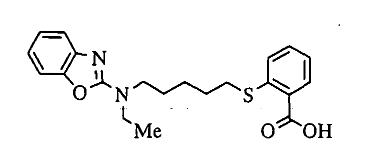

MS (m/z) 384 (M⁺)

### Example 100

### 2-[5-[N-(Benzoxazol-2-yl)-N-methyl]aminopentylthio]benzoic acid

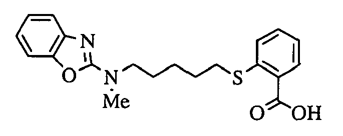

MS (m/z) 370 (M⁺)

### Example 101

### 2-[5-[N-(Benzoxazol-2-yl)-N-n-hexyl]aminopentylthio]benzoic acid

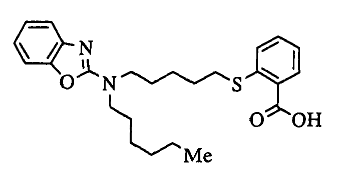

MS (m/z) 438 (M⁺)

### Example 102

### 2-[5-[N-(Benzoxazol-2-yl)-N-n-octyl]aminopentylthio]benzoic acid

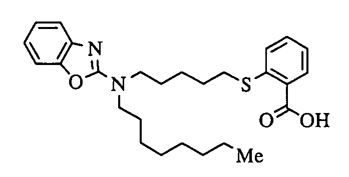

MS (m/z) 468 (M⁺)

### Example 103

### 2-[5-[N-(Benzoxazol-2-yl)-N-n-propyl]aminopentylthio]benzoic acid

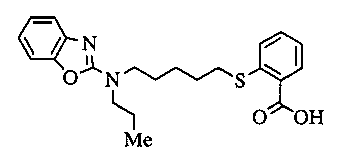

MS (m/z) 398 (M⁺)

### Example 104

### 2-[5-[N-(Benzoxazol-2-yl)-N-3-phenylpropyl]aminopentylthio]benzoic acid

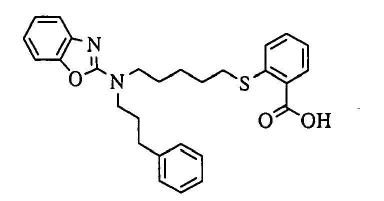

MS (m/z) 474 (M⁺)

### Example 105

### 2-[5-[N-(Benzoxazol-2-yl)-N-3-phenoxypropyl]aminopentylthio]benzoic acid

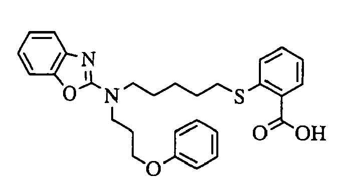

MS (m/z) 490 (M⁺)

### Example 106

### 2-[6-[N-(Benzoxazol-2-yl)-N-4-chlorobenzyl]aminohexylthio]benzoic acid

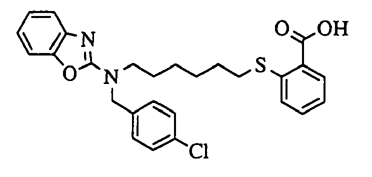

MS (m/z) 494 (M⁺), 496 (M⁺+2)

### Example 107

### 2-[4-[N-(Benzothiazol-2-yl)-N-3-(4-chlorophenoxy)propyl]aminobutylthio]benzoic acid

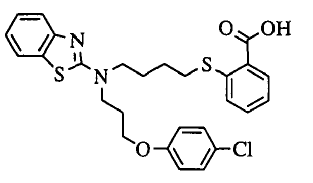

MS (m/z) 526 (M⁺), 528 (M⁺+2)

### Example 108

### 2-[4-[N-(Benzothiazol-2-yl)-N-ethyl]aminobutylthio]benzoic acid

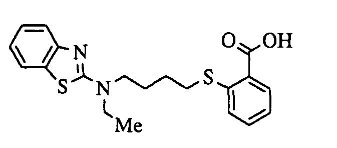

MS (m/z) 386 (M⁺)

### Example 109

### 2-[4-[N-(Benzothiazol-2-yl)-N-methyl]aminobutylthio] benzoic acid

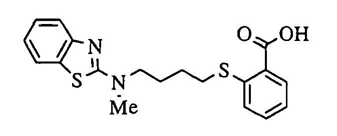

MS (m/z) 372 (M⁺)

### Example 110

### 2-[4-[N-(Benzothiazol-2-yl)-N-n-propyl]aminobutylthio]benzoic acid

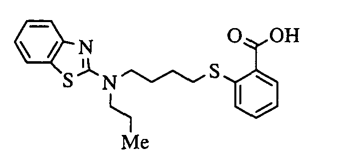

MS (m/z) 400 (M⁺)

### Example 111

### 2-[4-[N-(Benzothiazol-2-yl)-N-2-phenoxyethyl]aminobutylthio]benzoic acid

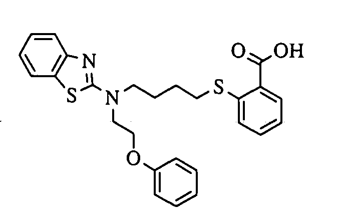

MS (m/z) 478 (M⁺)

### Example 112

### Synthesis of 2-[5-[N-(Benzoxazol-2-yl)-N-n-pentyl]aminopentylsulfonyl] benzoic acid

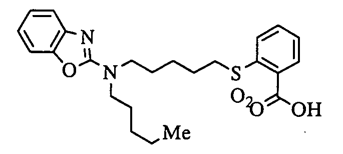

2-[5-[N-(Benzoxazol-2-yl)-N-n-pentyl]aminopentylthio]benz oic acid (34 mg, 0.08 mmol) obtained in Example 72 was dissolved in 10 mL of dichloromethane andreactedwithm-chloroperbenzoic acid (57 mg, 0.33 mmol) at 0°C. The resulting mixture was stirred at the same temperature for 2 hours and further stirred at room temperature. After the completion of the reaction, the resulting mixture was diluted with a sodium thiosulfate solution and subsequently with a methanol solution and extracted with chloroform. The organic layer was dried with sodium sulfate and then concentrated under reduced pressure, followed by purification by silica gel column chromatography (chloroform:methanol = 10:1) to give a compound of interest (28 mg, 75.9%).

¹H-NMR(270MHz, CD₃OD) δ:
0.90(t, J = 7Hz, 3H), 1.15-1.44(m, 6H), 1.62-1.74(m, 6H), 3.44(t, J = 7Hz, 4H), 3.66(t, J = 8Hz, 2H), 6.97(t, J = 8Hz, 1H), 7.11(t, J = 8Hz, 1H), 7.21-7.34(m, 2H), 7.39-7.51(m, 1H), 7.62(d, J = 3Hz, 2H), 7.92(d, J = 8Hz, 1H)
MS (m/z) 459 (M⁺)
Hereinafter, compounds of Examples 113 to 125 were synthesized in the same way as in Example 112.

### Example 113

### 2-[2-[N-(Benzoxazol-2-yl)-N-n-butyl]aminoethylsulfonyl]benzoic acid

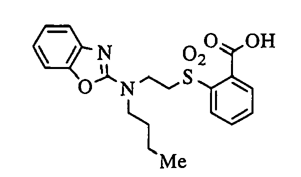

MS (m/z) 402 (M⁺)

### Example 114

### 2-[2-[N-(Benzoxazol-2-yl)-N-n-heptyl]aminoethylsulfonyl]benzoic acid

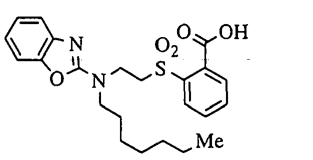

MS (m/z) 444 (M⁺)

### Example 115

### 2-[2-[N-(Benzoxazol-2-yl)-N-n-octyl]aminoethylsulfonyl]benzoic acid

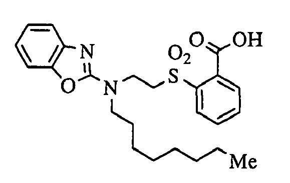

MS (m/z) 458 (M⁺)

### Example 116

### 2-[2-[N-(Benzoxazol-2-yl)-N-3-phenylpropyl]aminoethylsulfonyl]benzoic acid

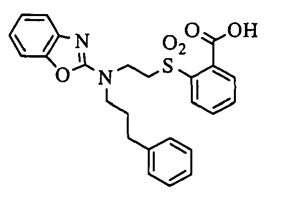

MS (m/z) 464 (M⁺)

### Example 117

### 2-[2-[N-(Benzoxazol-2-yl)-N-3-phenoxypropyl]aminoethylsulfonyl]benzoic acid

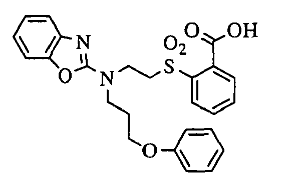

MS (m/z) 480 (M⁺)

### Example 118

### 2-[5-[N-(Benzoxazol-2-yl) N-4-chlorobenzyl]aminopentylsulfonyl]benzoic acid

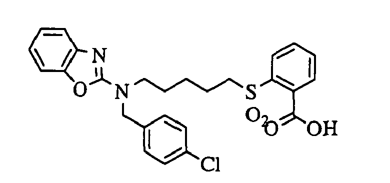

MS (m/z) 513 (M⁺), 515 (M⁺+2)

Example 119

### 2-[5-[N-(Benzoxazol-2-yl)-N-cyclohexylmethyl]aminopentylsulfonyl]benzoic acid

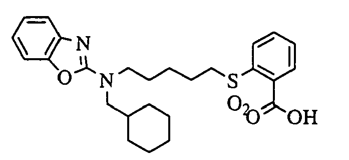

MS (m/z) 485 (M⁺)

### Example 120

### 2-[5-[N-(Benzoxazol-2-yl)-N-n-butyl]aminopentylsulfonyl]benzoic acid

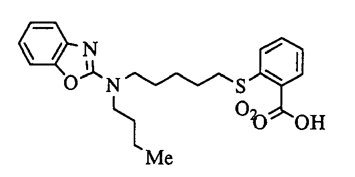

MS (m/z) 445 (M⁺)

### Example 121

### 2-[2-[N-(Benzoxazol-2-yl)-N-4-chlorobenzyl]aminoethylsulfonyl]benzoic acid

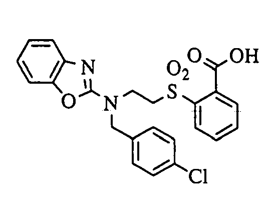

MS (m/z) 470 (M⁺), 472 (M⁺+2)

### Example 122

### 2-[5-[N-(Benzoxazol-2-yl)-N-n-octyl]aminopentylsulfonyl]benzoic acid

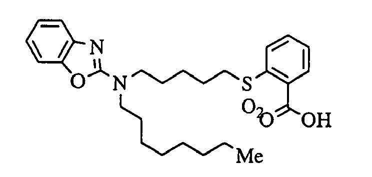

¹H-NMR (270MHz, CD₃OD) δ:
0.77 (t, J = 7Hz, 3H), 1.18-1.38 (m, 12H), 1.53-1.70 (m, 6H), 3.35 (t, J = 7Hz, 4H), 3.57 (t, J = 8Hz, 2H), 6.89 (t, J = 8Hz, 1H), 7.03 (t, J = 8Hz, 1H), 7.15 (t, J = 8Hz, 2H), 7.35-7.44 (m, 1H), 7.76 (d, J = 4Hz, 2H), 7.82 (d, J = 8Hz, 1H)
MS (m/z) 501 (M⁺)

### Example 123

### 2-[5-[N-(Benzoxazol-2-yl)-N-n-propyl]aminopentylsulfonyl]benzoic acid

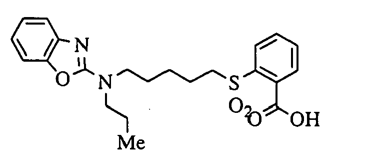

¹H-NMR (270MHz, CD₃OD) δ:
0.93 (t, J = 7Hz, 3H), 1.14-1.26 (m, 2H), 1.63-1.74 (m, 6H), 3.43 (dd, J = 14, 7Hz, 4H), 3.56-3.68 (m, 2H), 6.97 (t, J = 7Hz, 1H), 7.11 (t, J = 7Hz, 1H), 7.23 (t, J = 7Hz, 2H), 7.47-7.51 (m, 1H), 7.61 (d, J = 3Hz, 2H), 7.91 (d, J = 8Hz, 1H)

### Example 124

### 2-[5-[N-(Benzoxazol-2-yl)-N-3-phenylpropyl]aminopentylsulfonyl]benzoic acid

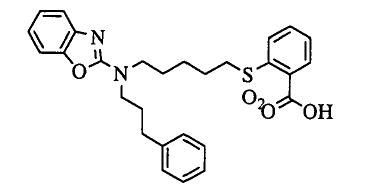

MS (m/z) 507 (M⁺)

### Example 125

### 2-[5-[N-(Benzoxazol-2-yl)-N-3-phenoxypropyl]aminopentylsulfonyl]benzoic acid

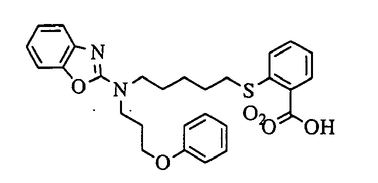

MS (m/z) 523 (M⁺)

### Test Example 1

The PPAR receptor-activating effect of the compound of the present invention represented by the formula (1) was measured by a method described below (Proc. Natl. Acad. Sci., 92, pp 7297-7301, 1995; Journal of Lipid Research, 40, pp 2099-2110, 1999; and Proc. Natl. Acad. Sci, 98, pp 5306-5311, 2001).

### (1) Measurement method

### Transfection assay

All the transfection assays were conducted using African greenmonkeykidney-derived cell line COS cells. The COS cells were cultured at 5% CO₂ concentration using a DMEM medium containing 10% fetal bovine serum, glutamic acid, and an antibiotic as a culture solution.
Gal4-human PPARs chimeric constructs in which the DNA-binding region of yeast transcription factor Gal4 was fused with the ligand-binding region of human PPARs were used as an expression vector. A chimera where lst to 147th amino acids of the Gal4 transcription factor were fused with 166th to 467th amino acids of human PPARα, with 182nd to 505th amino acids of human PPARγ2, or with 137th to 441st amino acids of human PPARδ was used as the chimera for each isoform. A reporter vector used was firefly luciferase whose promoter region contains five Gal4 recognition sequences. The plasmids were transfected into the cells by a method using Lipofectamine. In addition, a β-galactosidase expression vector was used as an internal standard.
After the transfection into the cells, the medium was substituted with a DMEM medium (containing 0.2% serum) supplemented with the compound, in which the cells were then cultured for additional 16 hours. Thereafter, luciferase activity and β-galactosidase activity in the cell lysate were measured.
In the present experiment, dimethyl sulfoxide (DMSO) was used in the dissolution and dilution of the compound. When the cells were treated, the DMSO concentration in the DMEM medium (containing 0.2% serum) was adjusted to 0.1%. Positive compounds used were WY14643 for PPARα, rosiglitazone (Journal of Medicinal Chemistry, 43, pp 527-550, 2000) for PPARγ, and GW501516 (Proc. Natl. Acad. Sci, 98, pp 5306-5311, 2001) for PPARδ.

### (2) Result

When luciferase activity at the addition of the positive compound for each isoform (PPARα: WY14643 10 µM, PPARγ: rosiglitazone 1 µM; and PPARδ: GW501516 1 µM) was given by 10.0, the relative activity of the compound of the present invention (added at 1 µM) was summarized in Table 1.

**Table 1-1**

| Example No. | hPPAR ; Relative activity | | |
|---|---|---|---|
| | α | γ | δ |
| 6 | 17.3 | 1.2 | 0.3 |
| 13 | 15.2 | 6.2 | 0.1 |
| 19. | 15.1 | 1.7 | 0.5 |
| 20 | 21.7 | 1.5 | 0.2 |
| 27 | 13.7 | 1.9 | 0.1 |
| 45 | 12.3 | 3.5 | 0.6 |
| 46 | 16.5 | 1.9 | 0.3 |
| 47 | 10.4 | 1.2 | 0.3 |
| 48 | 16.0 | 2.1 | 0.2 |
| 50 | 13.3 | 2.9 | 0.1 |
| 52 | 13.8 | 2.1 | 0.1 |
| 53 | 16.0 | 3.5 | 0.1 |
| 60 | 11.9 | 1.8 | 0.1 |
| 70 | 12.4 | 1.4 | 0.2 |
| 71 | 11.0 | 2.0 | 0.5 |
| 72 | 13.5 | 1.7 | 0.6 |
| 73 | 11.2 | 1.1 | 0.2 |
| 74 | 10.2 | 0.7 | 0.3 |
| 77 | 18.9 | 2.0 | 0.2 |
| 78 | 20.8 | 2.1 | 0.2 |
| 79 | 13.3 | 1.1 | 0.2 |
| 80 | 17.8 | 1.6 | 0.2 |
| 93 | 12.3 | 4.1 | 0.4 |
| 94 | 12.0 | 3.5 | 1.4 |
| 97 | 11.6 | 4.6 | 0.2 |
| 98 | 14.7 | 3.3 | 0.7 |
| 99 | 16.8 | 2.2 | 0.3 |
| 100 | 20.2 | 1.7 | 0.2 |

**Table 1-2**

| Example No. | hPPAR ; Relative activity | | |
|---|---|---|---|
| | α | γ | δ |
| 101 | 14.0 | 1.8 | 0.7 |
| 102 | 20.3 | 2.1 | 0.9 |
| 103 | 12.4 | 2.0 | 0.2 |
| 104 | 18.7 | 2.7 | 0.8 |
| 105 | 25.3 | 2.5 | 4.2 |
| 106 | 13.1 | 2.4 | 0.0 |
| 108 | 16.2 | 1.4 | 0.2 |
| 109 | 12.1 | 0.8 | 0.2 |
| 110 | 11.0 | 2.0 | 0.2 |
| 111 | 16.0 | 4.6 | 0.3 |
| 122 | 12.2 | 2.3 | 0.2 |
| WY14643 10 µM | 10.0 | - | - |
| Rosiglitazone 1 µM | - | 10.0 | - |
| GW501516 1 µM | - | - | 10.0 |

These results show that the compound of the present invention exhibits excellent hPPARα activation and selectivity. As described above, the compound of the present invention is an excellent hPPARα-selective activator.

## Claims

1. A benzoic acid derivative represented by the following general formula (1): 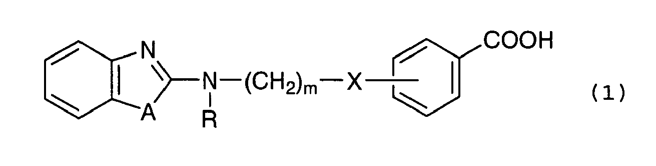 wherein A represents an oxygen atom, a nitrogen atom, or a sulfur atom;
R represents a hydrogen atom, a C₁₋₈ alkyl group, a C₃₋₈ alkenyl group, a C₃₋₈ alkynyl group, a C₃₋₇ cycloalkyl group, a C₃₋₇ cycloalkyl-C₁₋₈ alkyl group, a C₆₋₁₀ aryl group, a C₆₋₁₀ aryl-C₁₋₈ alkyl group (wherein the C₆₋₁₀ aryl moiety may be substituted with one or two selected from a halogen atom, a hydroxy group, a nitro group, an amino group, a di-C₁₋₄ alkylamino group, a C₁₋₄ alkyl group, a C₁₋₄ alkoxy group, a benzyloxy group, a phenylsulfonylmethyl group, and a C₁₋₄ alkanesulfonyloxy group), a C₆₋₁₀ aryl-oxy-C₁₋₈ alkyl group (wherein the C₆₋₁₀ aryl moiety may be substituted with one or two selected from a halogen atom, a hydroxy group, a nitro group, an amino group, a di-C₁₋₄ alkylamino group, a C₁₋₄ alkyl group, a C₁₋₄ alkoxy group, a benzyloxy group, a phenylsulfonylmethyl group, and a C₁₋₄ alkanesulfonyloxy group), a pyridyl-C₁₋₈ alkyl group, a C₁₋₈ alkoxycarbonyl-C₁₋₈ alkyl group, or a carboxy-C₁₋₈ alkyl group;
X represents an oxygen atom, an NH group, or an S(O)ₙ group (wherein n represents an integer of 0, 1, or 2); and
m represents an integer from 2 to 8; or a salt thereof.

2. The benzoic acid derivative or the salt thereof according to claim 1, wherein R represents a C₁₋₈ alkyl group, a C₃₋₇ cycloalkyl-C₁₋₈ alkyl group, a C₆₋₁₀ aryl group, a C₆₋₁₀ aryl-C₁₋₈ alkyl group (wherein the C₆₋₁₀ aryl moiety may be substituted with one or two selected from a halogen atom, a nitro group, and a C₁₋₄ alkyl group), or a C₆₋₁₀ aryl-oxy-C₁₋₈ alkyl group (wherein the C₆₋₁₀ aryl moiety may be substituted with a halogen atom).

3. A compound selected from 3- [3-[N-(Benzoxazol-2-yl)-N-3-(4-chlorophenoxy) ethyl]aminopropoxy]benzoic acid, 2-[5-[N-(Benzoxazol-2-yl)-N-isopropyl]aminopentylthio]benzoic acid, 2-[5-[N-(Benzoxazol-2-yl)-N-n-butyl]aminopentylthio]benzoic acid, 2-[5-[N-(Benzoxazol-2-yl)-N-n-pentyl]aminopentylthio]benzoic acid, and salts thereof.

4. A medicament comprising the benzoic acid derivative or a salt thereof according to any one of claims 1 to 3 as an active ingredient.

5. A preventive or therapeutic agent for hyperlipidemia comprising the benzoic acid derivative or a salt thereof according to any one of claims 1 to 3 as an active ingredient.

6. A preventive or therapeutic agent for arteriosclerosis comprising the benzoic acid derivative or a salt thereof according to any one of claims 1 to 3 as an active ingredient.

7. A preventive or therapeutic agent for diabetes comprising the benzoic acid derivative or a salt thereof according to any one of claims 1 to 3 as an active ingredient.

8. A preventive or therapeutic agent for diabetes complications comprising the benzoic acid derivative or a salt thereof according to any one of claims 1 to 3 as an active ingredient.

9. A preventive or therapeutic agent for inflammation comprising the benzoic acid derivative or a salt thereof according to any one of claims 1 to 3 as an active ingredient.

10. A preventive or therapeutic agent for cardiac diseases comprising the benzoic acid derivative or a salt thereof according to any one of claims 1 to 3 as an active ingredient.

11. A pharmaceutical composition comprising the benzoic acid derivative or a salt thereof according to any one of claims 1 to 3 and a pharmaceutically acceptable carrier.

12. Use of the benzoic acid derivative or a salt thereof according to any one of claims 1 to 3 for the manufacture of a preventive or therapeutic agent for hyperlipidemia.

13. Use of the benzoic acid derivative or a salt thereof according to any one of claims 1 to 3 for the manufacture of a preventive or therapeutic agent for arteriosclerosis.

14. Use of the benzoic acid derivative or a salt thereof according to any one of claims 1 to 3 for the manufacture of a preventive or therapeutic agent for diabetes.

15. Use of the benzoic acid derivative or a salt thereof according to any one of claims 1 to 3 for the manufacture of a preventive or therapeutic agent for diabetes complications.

16. Use of the benzoic acid derivative or a salt thereof according to any one of claims 1 to 3 for the manufacture of a preventive or therapeutic agent for inflammation.

17. Use of the benzoic acid derivative or a salt thereof according to any one of claims 1 to 3 for the manufacture of a preventive or therapeutic agent for cardiac diseases.
